# EUROPEAN PATENT SPECIFICATION

(11) **EP 1 401 852 B1**
(45) Date of publication and mention of the grant of the patent: **11.09.2013**
(21) Application number: 02756166.1
(22) Date of filing: 13.06.2002
(51) Int. Cl.: C07H 21/00, C07H 19/00

(54) **METHODS FOR PREPARING OLIGONUCLEOTIDES HAVING CHIRAL PHOSPHOROTHIOATE LINKAGES**
VERFAHREN ZUR HERSTELLUNG VON OLIGONUKLEOTIDEN MIT CHIRALEN PHOSPHOROTHIOATBINDUNGEN
PROCEDE D'OBTENTION D'OLIGONUCLEOTIDES PORTEURS DE LIAISONS PHOSPHOROTHIOATE CHIRALES

(30) Priority: 14.06.2001 US 881535
(43) Date of publication of application: 31.03.2004
(73) Proprietor: Isis Pharmaceuticals, Inc., Carlsbad, CA 92010 (US)
(72) Inventor: RAVIKUMAR, Vasulinga, T., Carlsbad, CA 92009 (US)
(74) Representative: Hallybone, Huw George
(86) International application number: PCT/US2002/018581
(87) International publication number: WO 2002/102815

(56) References cited:
- GUO M ET AL: "Solid-phase stereoselective synthesis of 2'-O-methyloligoribonucleos ide phosphorothioates using nucleoside bicyclic oxazaphospholidines" BIOORGANIC & MEDICINAL CHEMISTRY LETTERS, OXFORD, GB, vol. 8, no. 18, 22 September 1998 (1998-09-22), pages 2539-2544, XP004138266 ISSN: 0960-894X
- KATAOKA M ET AL: "IMIDAZOLIUM TRIFLATE AS AN EFFICIENT PROMOTER FOR O-SELECTIVE PHOSPHITYLATION OF N-UNPROTECTED NUCLEOSIDES VIA THE PHOSPHORAMIDITE APPROACH" NUCLEIC ACIDS SYMPOSIUM SERIES, IRL PRESS, OXFORD, GB, vol. 37, 1997, pages 21-22, XP008029228 ISSN: 0261-3166
- KROTZ A H ET AL: "On the Formation of Longmers in Phosphorothioate Oligodeoxyribonucleotide Synthesis" TETRAHEDRON LETTERS, ELSEVIER, AMSTERDAM, NL, vol. 38, no. 22, 2 June 1997 (1997-06-02), pages 3875-3878, XP004064869 ISSN: 0040-4039
- JIN ET AL.: 'Stereoselective synthesis of dithymidine phosphorothioates using xylose derivatives as chiral auxilaries' JOURNAL OF ORGANIC CHEMISTRY vol. 63, 1998, pages 3647 - 3654, XP002959212
- WANG ET AL.: 'A stereoselective synthesis of dinucleotide phosphorothioates, using chiral indol-oxazaphosphorine intermediates' TETRAHEDRON LETTERS vol. 38, no. 22, 1997, pages 3797 - 3800, XP004064849

## Description

### BACKGROUND

It is well known that most of the bodily states in multicellular organisms, including most disease states, are effected by proteins. Such proteins, either acting directly or through their enzymatic or other functions, contribute in major proportion to many diseases and regulatory functions in animals and man. For disease states, classical therapeutics has generally focused upon interactions with such proteins in efforts to moderate their disease-causing or disease-potentiating functions. In newer therapeutic approaches, modulation of the actual production of such proteins is desired. By interfering with the production of proteins, the maximum therapeutic effect can be obtained with minimal side effects. It is therefore a general object of such therapeutic approaches to interfere with or otherwise modulate gene expression, which would lead to undesired protein formation.

One method for inhibiting specific gene expression is with the use of oligonucleotides, especially oligonucleotides that are complementary to a specific target messenger RNA (mRNA) sequence. Phosphorothioate-linked nucleic acid analogs have found widespread application in therapeutic drug development and molecular biology. *See,* Crooke, S. T. in Antisense Therapeutics in Biotechnology & Genetic Engineering Reviews, 15, 1998, 121-157, Intercept Ltd, Hampshire, UK. The increased resistance to nuclease digestion displayed by these analogs has prompted their consideration for antisense therapy of a variety of diseases. *See,* Kisner, 12th International Roundtable on Nucleosides and Nucleotides, September 19, 1996, La Jolla, CA, USA. Several antisense phosphorothioate oligodeoxyribonucleotides (ODN) are currently undergoing clinical evaluation and the first antisense drug for treatment of CMV retinitis (Vitravene^{™}) has reached the market.

Successful use of oligonucleotides as drugs requires their stability *in vivo* long enough to be effective. The success of phosphorothioate modified oligonucleotides is due in part to the increased nuclease resistance of the phosphorothioate backbone relative to the naturally occurring phosphodiester backbone. The phosphorothioate linkage unlike the phosphodiester linkage has 2 enantiomers, R_{P} and S_{P}. Several groups have reported that the Rp isomer has enhanced binding properties to the target RNA and that the Sp isomer is significantly stable to exonucleases. (See Koziolkiewicz et al., Antisense & Nucleic acid drug development, 1997, 7, 43-8; Burgers et al., J. Biol. Chem., 1979, 254, 6889-93; and Griffiths et al., Nucleic Acids Research, 1987, 15, 4145-62). In fact, the degradation of a 25-mer having a 3'-Sp-terminal internucleotide linkage was calculated to be more than 300 times slower than an analog with a 3'-terminal R-configuration. *See,* Gilar, et al. Antisense & Nucleic Acid Drug Development, 8:35-42 (1998).

Current methods for preparing chiral phosphorothioate oligonucleotides involve synthesis and chromatographic isolation of stereoisomers of the chiral building blocks. (Stec et al., Angew. Chem. Int. Ed. Engl., 1994, 33, 709; Stec et al., J. Am. Chem. Soc., 1995, 117, 12019; and Stec WJ., Protocols for Oligonucleotides and Analogs: Synthesis and Properties, edited by Sudhir Agrawal, p. 63-80,(1993, Humana Press) and references cited therein). This method suffers from the non-stereospecific synthesis of the synthon. Recently, Just and coworkers presented the use of a chiral auxiliary to form dinucleotide phosphorothioate triesters in 97%ee (Wang, J.C., and Just G., Tetrahedron Letters, 1997, 38, 705-708). However, there was reported difficulty in removing the chiral auxiliary protecting group at phosphorous. This method has yet to be tested for convenient large-scale automated synthesis.

Stereoregular phosphorothioate analogs of pentadecamer 5'-d(AGATGTTTGA GCTCT)-3' were synthesized by the oxathiaphospholane method (Koziolkiewicz et al., Nucleic Acids Res., 1995, 23, 5000-5005). Enantiomeric purity was assigned by means of enzymic degradation with nuclease P1 and independently, with snake venom phosphodiesterase. DNA-RNA hybrids formed by phosphorothioate oligonucleotides (PS-oligos) with the corresponding complementary pentadecaribonucleotide were treated with bacterial RNase H. The DNA-RNA complex containing the [all-Rp] phosphorothioate oligomer was found to be more susceptible to RNase H-dependent degradation of the pentadecaribonucleotide compared with hybrids containing either the [all-Sp] counterpart or the so called random mixture of enantiomers of the pentadeca(nucleoside phosphorothioate). This stereodependence of RNase H action was also observed for a polyribonucleotide (475 nt) hybridized with these phosphorothioate oligonucleotides. The results of melting studies of PS-oligo-RNA hybrids allowed a rationalization of the observed stereodifferentiation in terms of the higher stability of heterodimers formed between oligoribonucleotides and [all-Rp]-oligo(nucleoside phosphorothioates), compared with the less stable heterodimers formed with [all-Sp]-oligo(nucleoside phosphorothioates) or the random mixture of enantiomers.

(S)-1-(indol-2-yl)-propan-2-ol was used as a chiral auxiliary to form a dinucleotide phosphorothioate triester in 97% ee (Wang et al., Tetrahedron Lett., 1997, 38,705-708).

A stereoselective preparation of dinucleotide phosphorothioates with a enantiomeric excess of > 98%, using hydroxy(indolyl)butyronitrile I as chiral auxiliaries, is reported (Wang et al., Tetrahedron Lett., 1997,38,3797-3800).

1,2-O-Cyclopentylidene-5-deoxy-5-isopropylamino-D-xylofuranose and its enantiomer were used as chiral auxiliaries to form, respectively, Sp and Rp dithymidine phosphorothioates in 98% enantiomeric excess, using phosphoramidite methodologies and 2-bromo-4,5-dicyanoimidazole as catalyst (Jin et al., J. Org. Chem., 1998,63,36473654).

Guo et al. (Bioorg. Med. Chem Lett. 1998, 8, 2539-2544) illustrates the use of chiral bicyclic phosphoramidites prepared from chiral chloro-oxazaphospholidine auxiliaries to influence the stereochemical outcome of the phosphorothioate linkage formation.

All of the prior methods require chemical and/or chromatographic steps in addition to those typically employed for large-scale oligonucleotide synthetic methods. Accordingly, a need in the art exists for simplified synthetic techniques for preparing phosphorothioate oligonucleotides having a predetermined chirality.

### SUMMARY OF THE INVENTION

According to one embodiment, the present invention provides methods for preparing an internucleotide phosphorothioate linkage that is enriched in the Rp enantiomer between a synthon having a hydroxyl moiety at the 5' position and a 2'-substituted nucleoside having an activated phosphate moiety at the 3'-position. The methods comprise selecting a coupling agent having a pKa ranging from about 3.3 to about 4.5 and coupling the synthon to said 2'-substituted nucleoside in the presence of the coupling agent. In some embodiments the agent has a pKa ranging from about 3.4 to about 4.4. A preferred range is from about 3.5 to about 4.3. A further preferred range is from about 3.6 to about 4.3. More preferably, the coupling agent has a pKa ranging from about 3.7 to about 4.3.

According to another embodiment, the present invention provides methods for preparing an internucleotide phosphorothioate linkage that is enriched in the Sp enantiomer between a synthon having a hydroxyl moiety at the 5' position and a 2'-substituted nucleoside having an activated phosphate moiety at the 3'-position. The methods comprise selecting a coupling agent having a pKa ranging from about 6.0 to about 7.5 and coupling the first synthon to the 2'- substituted nucleoside in the presence of the coupling agent. In preferred embodiments, the coupling agent has a pKa ranging from about 6.2 to about 7.3. In a further preferred embodiment, the coupling agent has a pKa ranging from about 6.4 to about 7.1. More preferably, the coupling agent has a pKa ranging from about 6.5 to about 7.0. A coupling agent having a pKa ranging from about 6.7 to about 6.9 is also contemplated.

In one embodiment of the present invention, methods for preparing an oligonucleotide having at least one region of internucleotide linkages that is enhanced in the Rp enantiomer are provided. Such methods comprise providing a nucleotide having a hydroxyl moiety at the 5'-position or a growing oligonucleotide chain having a hydroxyl moiety at the 5'-position and coupling the nucleotide or growing oligonucleotide chain to a 2'-substituted nucleoside having an activated phosphate moiety at the 3'-position in the presence of a coupling agent having a pKa ranging from about 3.3 to about 4.5. The coupling step is repeated until the desired number of linkages is established in the region. For example, an oligonucleotide having at least three Rp linkages at the 3'-end of the oligonucleotide is prepared by coupling the first four nucleotide subunits in the presence of a coupling agent having a pKa ranging from about 3.3 to about 4.5.

According to one embodiment, the oligonucleotide having at least one region of internucleotide linkages that is enhanced in the Rp enantiomer is further processed to include at least one region of internucleotide linkages that is enhanced in the Sp enantiomer by using a coupling agent having a pKa ranging from about 6.0 to about 7.5. In another embodiment, the oligonucleotide having at least one region of internucleotide linkages that is enhanced in the Sp enantiomer and having at least one region of internucleotide linkages that is enhanced in the Rp enantiomer is further processed to include another region of internucleotide linkages that is enhanced in the Sp enantiomer.

In a further embodiment, methods for preparing an oligonucleotide having at least one region of internucleotide linkgages that is enhanced in the Sp enantiomer are provided. These methods comprise providing a nucleotide having a hydroxyl moiety at the 5'-position or a growing oligonucleotide chain having a hydroxyl moiety at the 5'-position and coupling the nucleotide or growing oligonucleotide chain to a 2'-substituted nucleoside having an activated phosphate moiety at the 3'-position in the presence of a coupling agent having a pKa ranging from about 6.0 to 7.5 and repeating the coupling step until the desired number of linkages is established.

According to one embodiment, the oligonucleotide having at least one region of internucleotide linkages that is enhanced in the Sp enantiomer is further processed to include at least one region of internucleotide linkages that is enhanced in the Rp enantiomer by using a coupling agent having a pKa ranging from about 3.3 to about 4.5. In some embodiments, the oligonucleotide having at least one region of internucleotide linkages that is enhanced in the Rp enantiomer and having at least one region of internucleotide linkages that is enhanced in the Sp enantiomer is further processed to include another region of internucleotide linkages that is enhanced in the Rp enantiomer.

Thus, according to the present invention, it is possible to prepare oligonucleotides having defined regions of chirality by choosing a coupling agent having a pKa that will enhance the internucleotide linkages in either the Rp or Sp enantiomer.

### BRIEF DESCRIPTION OF THE DRAWINGS

The present invention will be better understood by reference to the Figures, in which,
Figure 1 shows an example of a typical phosphoramidite coupling scheme for oligonucleotide synthesis;

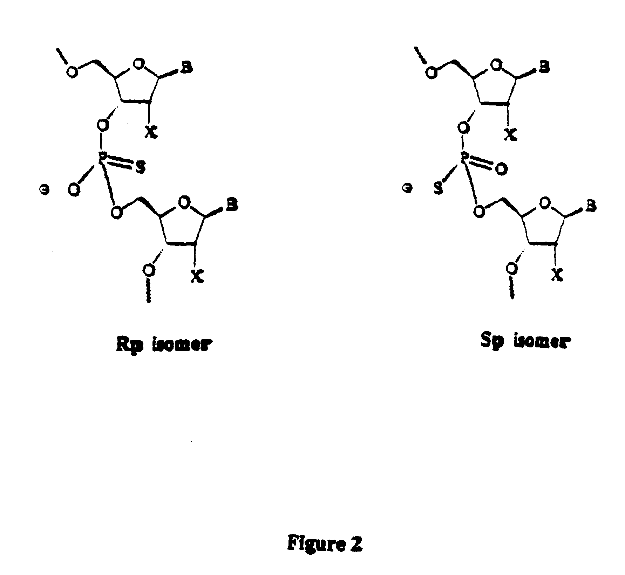

Figure 2 shows two representative examples of an Rp and an Sp internucleotide linkage;
Figure 3 represents a ³¹P NMR spectroscopy of two enantiomers of 5'-*O*-DMT-*N*²-isobutyryl-2'-*O*-methoxyethylguanosine-3'-*O*-(2-cyanoethyl) phosphoramidite; and
Figure 4 is represents a ³¹P NMR spectroscopy of a monophosphorothioate linkage in a 1:1 enantiomeric ratio.

The present invention is directed to methods for preparing an internucleotide phosphorothioate linkage enriched in either the Rp or Sp enantiomer. These methods are useful for, *inter alia,* synthesizing oligonucleotides having predetermined chirality wherein such chiral linkages possess relatively high enantiomeric purity. As will be recognized by those skilled in the art, enantiomeric purity -- also known as chiral purity - - is manifested for a chemical compound by the predominance of one enantiomer over the other. Thus, an oligonucleotide can be said to possess a substantially pure chiral phosphate linkage where, for example, the Sp form of that linkage greatly predominates over the Rp form. In accordance with the present invention, at least certain of the chiral phosphate linkages present in an oligonucleotide should have chiral purity greater than about 60%. Preferably such linkages have chiral purity greater than about 70%, more preferably greater than about 90%, even more preferably about 100%. Chiral purity may be determined by any of the many methods known in the art, including but not limited to x-ray diffraction, optical rotary dispersion, and circular dichroism.

The methods of the present invention include coupling a synthon having a hydroxyl moiety at the 5' position and a 2'-substituted nucleoside having an activated phosphate moiety at the 3'-position in the presence of a coupling agent that is selected to enhance the R to S ratio to provide linkages that are enriched in one of the Rp or Sp enantiomers. Although not wishing to be bound by any particular theory, it has been found that the pKa of the coupling agent influences the enantiomeric ratio of Sp to Rp linkages when the coupling agents are used in the methods of the present invention. For example, according to one embodiment of the present invention, coupling agents having a pKa ranging from about 3.3 to about 4.5 provide internucleotide linkages that are enriched in the Rp enantiomer. In another embodiment of the present invention, coupling agents having a pKa ranging from about 6.0 to about 7.5 provide internucleotide linkages that are enriched in the Sp enantiomer.

To enrich a phosphorothioate linkage in the Rp enantiomer, a coupling agent such as 5-(ethylthio)-1*H-*tetrazole is preferable, which has a pKa of about 4.3. To enrich the phosphorothioate linkage in the Sp enantiomer, coupling agents such as imidazolium derivatives are preferred. For example, imidazolium salts such imidazolium trifluoroacetate, imidazolium triflate, imidazolium perchlorate, imidazolium acetate, imidazolium tosylate or imidazolium nitrate are preferred. The use of imidazolium triflate as a coupling agent is well known in the art *See, e.g.* Nucleic Acid Symposium Series No. 37, 21-22 (Oxford Press 1997). Other coupling agents that are amenable to the present invention are within the purview of the art skilled and are not limited to those disclosed herein.

The pKa is determined using methods that are well known in the art such as potentiometric titrations. Cookson, R. F. Chemical Reviews, Vol. 74, No. 1, page 5 (1972) discusses various methods for pKa determination, the disclosure of which is herein incorporated by reference.

The nucleosides of the present invention include naturally and non-naturally occurring nucleosides. As used herein, the term "nucleoside" refers to a sugar and a nucleobase that are joined together, normally about an "anomeric" carbon on the sugar. Non-naturally occurring nucleosides and nucleotides may be modified by replacing the sugar moiety with an alternative structure having primary and secondary alcohol groups similar to those of ribose. Non-naturally occurring sugars and nucleosidic bases are typically structurally distinguishable from, yet functionally interchangeable with, naturally occurring sugars (*e.g.* ribose and deoxyribose) and nucleosidic bases (e.g., adenine, guanine, cytosine, thymine). Thus, non-naturally occurring nucleobases and sugars include all such structures which mimic the structure and/or function of naturally occurring species, and which aid in the binding of the oligonucleotide to a target, or which otherwise advantageously contribute to the properties of the oligonucleotide.

A heterocyclic base moiety (often referred to in the art simply as a "base" or a "nucleobase") amenable to the present invention includes both naturally and non-naturally occurring nucleobases. The heterocyclic base moiety further may be protected wherein one or more functionalities of the base bears a protecting group. As used herein, "unmodified" or "natural" nucleobases include the purine bases adenine and guanine, and the pyrimidine bases thymine, cytosine and uracil. Modified nucleobases include other synthetic and natural nucleobases such as 5-methylcytosine (5-me-C), 5-hydroxymethyl cytosine, xanthine, hypoxanthine, 2-aminoadenine, 6-methyl and other alkyl derivatives of adenine and guanine, 2-propyl and other alkyl derivatives of adenine and guanine, 2-thiouracil, 2-thiothymine and 2-thiocytosine, 5-halouracil and cytosine, 5-propynyl uracil and cytosine, 6-aza uracil, cytosine and thymine, 5-uracil (pseudouracil), 4-thiouracil, 8-halo, 8-amino, 8-thiol, 8-thioalkyl, 8-hydroxyl and other 8-substituted adenines and guanines, 5-halo particularly 5-bromo, 5-trifluoromethyl and other 5-substituted uracils and cytosines, 7-methylguanine and 7-methyladenine, 8-azaguanine and 8-azaadenine, 7-deazaguanine and 7-deazaadenine and 3-deazaguanine and 3-deazaadenine. Further nucleobases include those disclosed in United States Patent No. 3,687,808, those disclosed in the Concise Encyclopedia Of Polymer Science And Engineering, pages 858-859, Kroschwitz, J.I., ed. John Wiley & Sons, 1990, those disclosed by Englisch et al., Angewandte Chemie, International Edition, 1991, 30, 613*,* and those disclosed by Sanghvi, Y.S., Chapter 15, Antisense Research and Applications, pages 289-302, Crooke, S.T. and Lebleu, B., ed., CRC Press, 1993.

Certain heterocyclic base moieties are particularly useful for increasing the binding affinity of the oligomeric compounds of the invention to complementary targets. These include 5-substituted pyrimidines, 6-azapyrimidines and N-2, N-6 and O-6 substituted purines, including 2-aminopropyladenine, 5-propynyluracil and 5-propynylcytosine. 5-methylcytosine substitutions have been shown to increase nucleic acid duplex stability by 0.6-1.2 °C (*Id.,* pages 276-278) and are presently preferred base substitutions, even more particularly when combined with selected 2'-sugar modifications such as 2'-methoxyethyl groups.

Representative United States patents that teach the preparation of heterocyclic base moieties (modified nucleobases) include, but are not limited to, U.S. Patents 3,687,808; 4,845,205; 5,130,302; 5,134,066; 5,175,273; 5,367,066; 5,432,272; 5,457,187; 5,459,255; 5,484,908; 5,502,177; 5,525,711; 5,552,540; 5,587,469; 5,594,121, 5,596,091; 5,614,617; and 5,681,941, certain of which are commonly owned, and each of which is herein incorporated by reference, and commonly owned United States patent application 08/762,587, filed on December 10, 1996, also herein incorporated by reference.

"Activated phosphate moiety" refers to activated monomers that are reactive with a hydroxyl group of another monomeric or oligomeric compound (synthon) to form a phosphorus-containing internucleotide linkage. Such activated phosphorus groups contain activated phosphorus atoms in P^{III} valency states, such as phosphoramidites, which are well known in the art. The intermediate phosphite compounds are subsequently oxidized to the P^{V} state using known methods to yield, in a preferred embodiment, phosphorothioate internucleotide linkages.

A representative list of nucleosides having activated phosphate moieties include those having the formula: 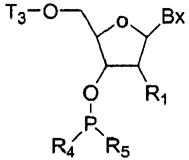 wherein
each Bx is, independently, a heterocyclic base moiety or a blocked heterocyclic base moiety; and
R₁ is a substituent group, or a blocked substituent group;
T₃ is an hydroxyl protecting group;
R₄ is N(L₁)L_{2;}
each L₁ and L₂ is, independently, C₁₋₆ alkyl;
R₅ is X₁;
X₁ is Pg-O-, Pg-S-, C₁-C₁₀ straight or branched chain alkyl, CH₃(CH₂)ₙₙ-O- or R₂R₃N-;
Pg is a phosphorus blocking group.

"Phosphorus blocking group" refers to a group that is initially bound to the phosphorus atom of a phosphoramidite. The phosphorus blocking group functions to protect the phosphorus containing internucleotide linkage or linkages during, for example, solid phase oligonucleotide synthetic regimes. Treatment of the internucleotide linkage or linkages that have a phosphorus blocking group thereon with a deblocking agent, such as aqueous ammonium hydroxide, will result in the removal of the phosphorus blocking group and leave a hydroxyl or thiol group in its place.

There are many phosphorus blocking groups known in the art which are useful in the present invention including, but not limited, to β-cyanoethyl, diphenylsilylethyl, δ-cyanobutenyl, cyano *p*-xylyl (CPX), methyl-N-trifluoroacetyl ethyl (META) and acetoxy phenoxy ethyl (APOE) groups. Phosphorus protecting groups are further described in Beaucage, S.L. and Iyer, R.P., Tetrahedron, 1993, 49, 1925-1963; Beaucage, S.L. and Iyer, R.P., Tetrahedron, 1993, 49, 10441-10488; and Beaucage, S.L. and Iyer, R.P., Tetrahedron, 1992, 48, 2223-2311. Representative United States patents that teach the preparation of phosphorus protecting groups and their incorporation into phosphoramidite compounds include, but are not limited to, United States Patent Nos. 5,783,690; 5,760,209; 5,705621; 5,614,621; 5,453,496; 5,153,319; 5,132,418; 4,973,679; 4,725,677; 4,668,777; 4,500,707; 4,458,066; 4,415,732; and Re. 34,069, the entire contents of each of which are herein incorporated by reference.

The methods of the present invention require 2'- substituents on the incoming nucleosides. Also, the synthon nucleosides or nucleotides may include 2'-substituents. The substituents at the 2'-position include those that are well known in the art for improving the properties of the oligonucleotide, for example, C₁-C₂₀ alkyl, C₂-C₂₀ alkenyl, C₂-C₂₀ alkynyl, C₅-C₂₀ aryl, O-alkyl, O-alkenyl, O-alkynyl, O-alkylamino, O-alkylalkoxy, O-alkylaminoalkyl, O-alkyl imidazole, S-alkenyl, S-alkynyl, NH-alkyl, NH-alkenyl, NH-alkynyl, N-dialkyl, O-aryl, S-aryl, NH-aryl, O-aralkyl, S-aralkyl, NH-aralkyl, N-phthalimido, halogen (particularly fluoro), keto, carboxyl, nitro, nitroso, nitrile, trifluoromethyl, trifluoromethoxy, imidazole, azido, hydrazino, hydroxylamino, isocyanato, sulfoxide, sulfone, sulfide, disulfide, silyl, heterocycle, carbocycle, polyamine, polyamide, polyalkylene glycol, and polyethers of the formula (O-alkyl)ₘ, where m is 1 to about 10. Preferred among these polyethers are linear and cyclic polyethylene glycols (PEGs), and (PEG)-containing groups, such as crown ethers and those which are disclosed by Ouchi et al. (Drug Design and Discovery 1992, 9, 93), Ravasio et al. (J. Org. Chem. 1991, 56, 4329) and Delgardo et. al. (Critical Reviews in Therapeutic Drug Carrier Systems 1992, 9, 249), each of which is herein incorporated by reference in its entirety. Further sugar modifications are disclosed in Cook, P.D., Anti-Cancer Drug Design, 1991, 6, 585-607. Fluoro, O-alkyl, O-alkylamino, O-alkyl imidazole, O-alkylaminoalkyl, and alkyl amino substitution is described in United States Patent Application serial number 08/398,901, filed March 6, 1995, entitled Oligomeric Compounds having Pyrimidine Nucleotide(s) with 2' and 5' Substitutions, hereby incorporated by reference in its entirety.

Additional substituent groups amenable to the present invention include -SR and -NR₂ groups, wherein each R is, independently, hydrogen, a protecting group or substituted or unsubstituted alkyl, alkenyl, or alkynyl. 2'-SR nucleosides are disclosed in United States Patent No. 5,670,633, issued September 23, 1997, hereby incorporated by reference in its entirety. The incorporation of 2'-SR monomer synthons are disclosed by Hamm et al., J. Org. Chem., 1997, 62, 3415-3420. 2'-NR₂ nucleosides are disclosed by Goettingen, M., J. Org. Chem., 1996, 61, 73-6281; and Polushin et al., Tetrahedron Lett., 1996, 37, 3227-3230.

Further substituent groups have one of formula I or II: 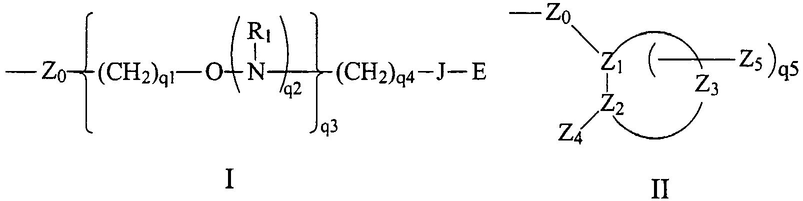 wherein:
Z₀ is O, S or NH;
J is a single bond, O or C(=O);
E is C₁-C₁₀ alkyl, N(R₁)(R₂), N(R₁)(R₅), N=C(R₁)(R₂), N=C(R₁)(R₅) or has one of formula III or IV; 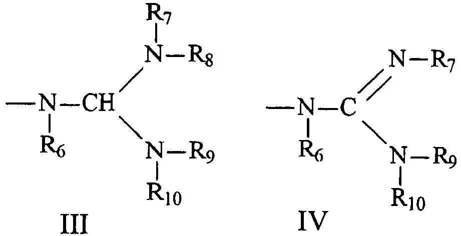
each R₆, R₇, R₈, R₉ and R₁₀ is, independently, hydrogen, C(O)R₁₁, substituted or unsubstituted C₁-C₁₀ alkyl, substituted or unsubstituted C₂-C₁₀ alkenyl, substituted or unsubstituted C₂-C₁₀ alkynyl, alkylsulfonyl, arylsulfonyl, a chemical functional group or a conjugate group, wherein the substituent groups are selected from hydroxyl, amino, alkoxy, carboxy, benzyl, phenyl, nitro, thiol, thioalkoxy, halogen, alkyl, aryl, alkenyl and alkynyl;
or optionally, R₇ and R₈, together form a phthalimido moiety with the nitrogen atom to which they are attached;
or optionally, R₉ and R₁₀, together form a phthalimido moiety with the nitrogen atom to which they are attached;
each R₁₁ is, independently, substituted or unsubstituted C₁-C₁₀ alkyl, trifluoromethyl, cyanoethyloxy, methoxy, ethoxy, t-butoxy, allyloxy, 9-fluorenylmethoxy, 2-(trimethylsilyl)-ethoxy, 2,2,2-trichloroethoxy, benzyloxy, butyryl, iso-butyryl, phenyl or aryl;
R₅ is T-L,
T is a bond or a linking moiety;
L is a chemical functional group, a conjugate group or a solid support material;
each R₁ and R₂ is, independently, H, a nitrogen protecting group, substituted or unsubstituted C₁-C₁₀ alkyl, substituted or unsubstituted C₂-C₁₀ alkenyl, substituted or unsubstituted C₂-C₁₀ alkynyl, wherein said substitution is OR₃, SR₃, NH₃⁺, N(R₃)(R₄), guanidino or acyl where said acyl is an acid amide or an ester;
or R₁ and R₂, together, are a nitrogen protecting group or are joined in a ring structure that optionally includes an additional heteroatom selected from N and O;
or R₁, T and L, together, are a chemical functional group;
each R₃ and R₄ is, independently, H, C₁-C₁₀ alkyl, a nitrogen protecting group, or R₃ and R₄, together, are a nitrogen protecting group;
or R₃ and R₄ are joined in a ring structure that optionally includes an additional heteroatom selected from N and O;
Z₄ is OX, SX, or N(X)₂;
each X is, independently, H, C₁-C₈ alkyl, C₁-C₈ haloalkyl, C(=NH)N(H)R₅, C(=O)N(H)R₅ or OC(=O)N(H)R₅;
R₅ is H or C₁-C₈ alkyl;
Z₁, Z₂ and Z₃ comprise a ring system having from about 4 to about 7 carbon atoms or having from about 3 to about 6 carbon atoms and 1 or 2 hetero atoms wherein said hetero atoms are selected from oxygen, nitrogen and sulfur and wherein said ring system is aliphatic, unsaturated aliphatic, aromatic, or saturated or unsaturated heterocyclic;
Z₅ is alkyl or haloalkyl having 1 to about 10 carbon atoms, alkenyl having 2 to about 10 carbon atoms, alkynyl having 2 to about 10 carbon atoms, aryl having 6 to about 14 carbon atoms, N(R₁)(R₂) OR₁, halo, SR₁ or CN;
each q₁ is, independently, an integer from 1 to 10;
each q₂ is, independently, 0 or 1;
q₃ is 0 or an integer from 1 to 10;
q₄ is an integer from 1 to 10;
q₅ is from 0, 1 or 2; and
provided that when q₃ is 0, q₄ is greater than 1.

Representative substituent groups of Formula I are disclosed in United States Patent Application Serial No. 09/130,973, filed August 7, 1998, entitled "Capped 2'-Oxyethoxy Oligonucleotides," hereby incorporated by reference in its entirety.

Representative cyclic substituent groups of Formula II are disclosed in United States Patent Application Serial No. 09/123,108, filed July 27, 1998, entitled "RNA Targeted 2'-Modified Oligonucleotides that are Conformationally Preorganized," hereby incorporated by reference in its entirety.

Particularly preferred substituent groups include O[(CH₂)ₙO]ₘCH₃, O(CH₂)ₙOCH₃, O(CH₂)ₙNH₂, O(CH₂)ₙCH₃, O(CH₂)ₙONH₂, O(CH₂)ₙON[(CH₂)ₙCH₃)]₂ (where n and m are from 1 to about 10), C₁ to C₁₀ lower alkyl, substituted lower alkyl, alkaryl, aralkyl, O-alkaryl or O-aralkyl, SH, SCH₃, OCN, Cl, Br, CN, CF₃, OCF₃, SOCH₃, SO₂CH₃, ONO₂, NO₂, N₃, NH₂, heterocycloalkyl, heterocycloalkaryl, aminoalkylamino, polyalkylamino and substituted silyl. Another particularly preferred modification includes 2'-methoxyethoxy (2'-O-CH₂CH₂OCH₃ or 2'-MOE, Martin et al., Helv. Chim. Acta, 1995, 78, 486). A further preferred substituent group is 2'-dimethylaminooxyethoxy, *i.e*., a O(CH₂)₂ON(CH₃)₂ group, also known as 2'-DMAOE. Representative aminooxy substituent groups are described in co-owned United States Patent Application serial number 09/344,260, filed June 25, 1999, entitled "Aminooxy-Functionalized Oligomers"; and United States Patent Application serial number 09/370,541, filed August 9, 1999, also identified by attorney docket number ISIS-3993, entitled Aminooxy-Functionalized Oligomers and Methods for Making Same; hereby incorporated by reference in their entirety.

Other preferred modifications include 2'-methoxy (2'-O-CH₃), 2'-aminopropoxy (2'-OCH₂CH₂CH₂NH₂) and 2'-fluoro (2'-F). The configuration of the substituent group is also variable such as at the 2'-position. In addition to the ribose configuration, the arabinose configuration is also amenable to the present invention. Arabinose modifications are known to those skilled in the art and include more recent procedures described in for example, Damha et. al., J.A.C.S., 1998, 120, 12976-12977; Bioconjugate Chem., 1999, 10, 299-305; Nucleic Acids Res. (2000), 28(18), 3625-3635; Biochemistry (2000), 39(24), 7050-7062.

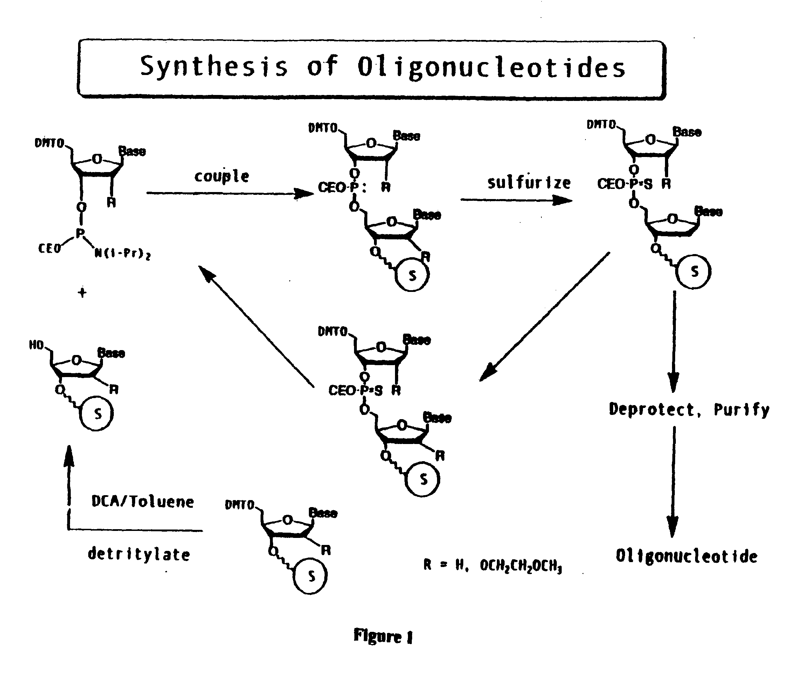

Referring to Figure 1, according to one embodiment, phosphorothioate linkages are prepared wherein the 3'-O-position of the synthon is coupled to a support. The synthon is a first nucleoside or a nucleotide synthon, which is then iteratively elongated to give a final oligomeric compound. Support media can be selected to be insoluble or have variable solubility in different solvents to allow the growing oligomer to be kept out of or in solution as desired. Traditional solid supports are insoluble and are routinely placed in a reaction vessel while reagents and solvents react and or wash the growing chain until cleavage frees the final oligomer. More recent approaches have introduced soluble supports including soluble polymer supports to allow precipitating and dissolving the bound oligomer at desired points in the synthesis (Gravert et al., Chem. Rev., 1997, 97, 489-510). Representative support media that are amenable to the methods of the present invention include without limitation: controlled pore glass (CPG); oxalyl-controlled pore glass (*see, e.g.,* Alul, et al., Nucleic Acids Research 1991, 19, 1527); TENTAGEL Support, (*see, e.g.,* Wright, et al., Tetrahedron Letters 1993, 34, 3373); or POROS, a copolymer of polystyrene/divinylbenzene available from Perceptive Biosystems. The use of a soluble support media, poly(ethylene glycol), with molecular weights between 5 and 20 kDa, for large-scale synthesis of phosphorothioate oligonucleotides is described in, Bonora et al., Organic Process Research & Development, 2000, 4, 225-231.

Phosphoramidite coupling is the current route of choice for large-scale synthesis of uniform phosphorothioate oligodeoxyribonucleotides because of its potential for automation, high coupling efficiency, and ready scaleability. See, Beaucage and Iyer, Tetrahedron, 1992, 24, 2223 and references cited therein; Iyer, R. P.; Beaucage, S. L. In Comprehensive Natural Products Chemistry, (Eds.: Barton, D. H. R.; Nakanishi, K.) in Volume 7: DNA and Aspects of Molecular Biology, Ed. Kool, E. T., 1999, 105, Pergamon Press. Typically, oligonucleotide synthesis on scales up to 200 mmoles is performed in a cyclic manner on the Pharmacia OligoProcess™ DNA/RNA synthesizer using a packed-bed column. Deoxyribonucleoside phosphoramidite coupling is highly efficient at low synthon excess (1.75 - 2.0 molar equivalents) and coupling efficiency is very high (98.5 - 98.7%). The total synthesis cycle time is short (< 8 h) for a 20-mer phosphorothioate.

The phosphoramidite approach for the automated synthesis of phosphorothioate analogs of DNA and modified RNA involves repetitive formation of chiral phosphite triester intermediates, followed by oxidative sulfurization to the phosphorothioate triester linkages. Although sulfurization can be accomplished using a variety of sulfur-transfer reagents including 3*H*-1,2-benzodithiol-3-one 1,1-dioxide, as described by Iyer, et al.. J. Org. Chem. 1990, 55, 4693 and Iyer, et al J. Am. Chem. Soc. 1990, 112, 1253, phenylacetyl disulfide (PADS) has become very popular for the development of antisense drugs due to its very high sulfurization efficiency and inexpensive nature. See, Cheruvallath, et al. Org. Process Res. Dev., 2000, 4, 199-204 and Cheruvallath, et al. Nucleosides Nucleotides, 1999, 18, 1195-1197.

There are several reports in the literature which show that 1*H*-tetrazole-activated deoxynucleoside phosphoramidite coupling is a racemization process leading to a 1:1 mixture of Rp and Sp phosphorothioate enantiomers even if one starts with 100% enantiomerically pure phosphoramidite. It has been hypothesized that if chiral atoms or relatively bulky groups like 2'-*O*-methoxyethyl are present in the vicinity of the phosphorous center, the stereochemical outcome of the coupling reaction could be influenced substantially.

A systematic evaluation of the various plausible contributors to the stereochemistry of phosphorothioate internucleotide linkages reveals that the Rp to Sp ratios are reproducible between syntheses and independent of enantiomeric composition of the starting material (phosphoramidites), synthesis scale, solid support, reactor or synthesis conditions when coupling agents such as 1*H*-tetrazole and 4,5-dicyanoimidazole (DCI) and pyridinium trifluoroacetate (PTFA) are employed.

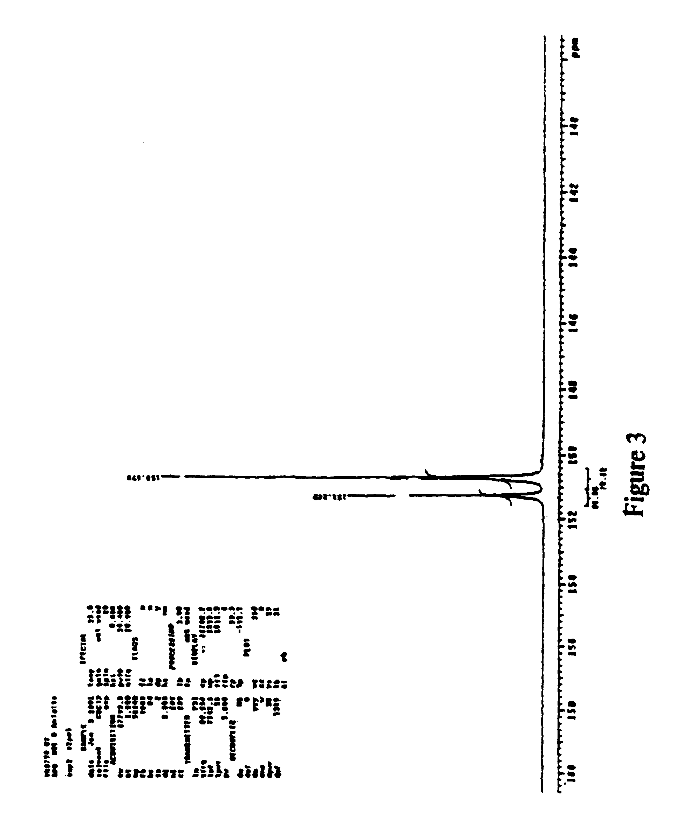

Enantiomeric compositition of phosphoramidites was investigated to determine whether it is a factor contributing to final chirality of phosphorothioate linkages. In order to determine whether 1*H*-tetrazole-activated coupling of 2'-*O*-methoxyethyl-3'-*O*-(2-cyanoethyl) phosphoramidites is a racemization process the ratio of two enantiomers of commercially available 5'-*O*-DMT-*N*²-isobutyryl-2'-*O*-methoxyethylguanosine-3'-*O*-(2-cyanoethyl) phosphoramidite by ³¹P NMR spectroscopy (Figure 3) was investigated. Analysis of the spectrum reveals that the two enantiomers are present in the approximate ratio of 3:1. Hence, the ratio of the two enantiomers was not further enriched.

Short oligonucleotides about 5-mer in length were synthesized in which a single phosphorus center was replaced by a phosphorothioate linkage. Oligonucleotides were synthesized using HL-30 thymidine Primer Support on the OligoPilot II DNA/RNA synthesizer using phenylacetyl disulfide as the sulfurizing agent. The DMT group on the final base at the 5' terminus was removed on the synthesizer column. After standard deprotection (30% concentrated ammonium hydroxide, 55°C) the oligonucleotide was analyzed by RP-HPLC. ³¹P NMR was used for analysis of the phosphorothioate linkages. Good separation of the two enantiomer signals was observed. A minimum signal-to-noise ratio of 200 was obtained for all samples analyzed. Table 1 shows the ratios of the two enantiomers obtained using the 2'-*O*-methoxyethylguanosine phosphoramidite.

**Table 1. Analysis of 5-mers using enantiomerically enriched 2'-O-MOE G amidite**

| **Expt** # | **Oligomer** | **Scale (µmole)** | ³¹P **NMR (D₂O)** | |
|---|---|---|---|---|
| | | | **ppm (Rp/Sp)** | **Enantiomer Ratio** |
| 750-84 | 5'-MOE G ps MOE U^{me} po TTT-3' | 148 | 58.22 / 56.02 | 49.02 / 50.98 |
| 750-85 | 5'-MOE G ps MOE U^{me} po TTT-3' | 158 | 58.25 / 56.01 | 49.17 / 50.83 |
| 750-86 | 5'-MOE G ps MOE U^{me} po TTT-3' | 147 | 58.26 / 56.02 | 49.33 / 50.67 |
| 750-87 | 5'-MOE G ps MOE U^{me} po TTT-3' | 140 | 58.21 / 56.01 | 49.46 / 50.54 |
| | | | | |
| 750-88 | 5'-MOE G ps TTTT-3' | 149 | 57.69 / 56.32 | 49.17 / 50.83 |
| 750-89 | 5'-MOE G ps TTTT-3' | 148 | 57.68 / 56.32 | 49.27 / 50.73 |
| 750-90 | 5'-MOE G ps'TTTT-3' | 154 | 57.68 / 56.33 | 49.55 / 50.45 |
| 750-91 | 5'-MOE G ps TTTT-3' | 160 | 57.68 / 56.32 | 49.68 / 50.32 |
| | | | | |
| 750-92 | 5'-MOE G ps MOE C^{me} po TTT-3' | 149 | 59.52 / 55.31 | 49.65 / 50.35 |
| 750-93 | 5'-MOE G ps MOE C^{me} po TTT-3' | 151 | 59.48 / 55.36 | 49.99 / 50.01 |
| 750-94 | 5'-MOE G ps MOE C^{me} po TTT-3' | 147 | 59.51 / 55.31 | 49.39 / 50.61 |
| 750-95 | 5'-MOE G ps MOE C^{me} po TTT-3' | 162 | 59.56 / 55.23 | 49.71 / 50.29 |

**Assignment of Stereochemistry:** The *Rp* and *Sp* configurations of the 2'-*O-*methoxyethyl modified phosphorothioate linkages were tentatively assigned based on earlier investigation of an analogous molecule viz. 2'-*O*-methyl oligoribonucleotide phosphorothioates. Guo et al., Bioorg. Med. Chem. Lett., 1998, 8, 2539-2544; Iyer et al. Agrawal, S. Tetrahedron Lett., 1998, 39, 2491-2494; Iyer et al. Bioorg. Med. Chem. Lett., 1994, 4, 2471-2474. Thus, the upfield shift in the ³¹P NMR signal of the 2'-*O-*methoxyethyl modified phosphorothioate diester linkage was assigned the Sp configuration and the downfield shift signal was assigned the Rp configuration.

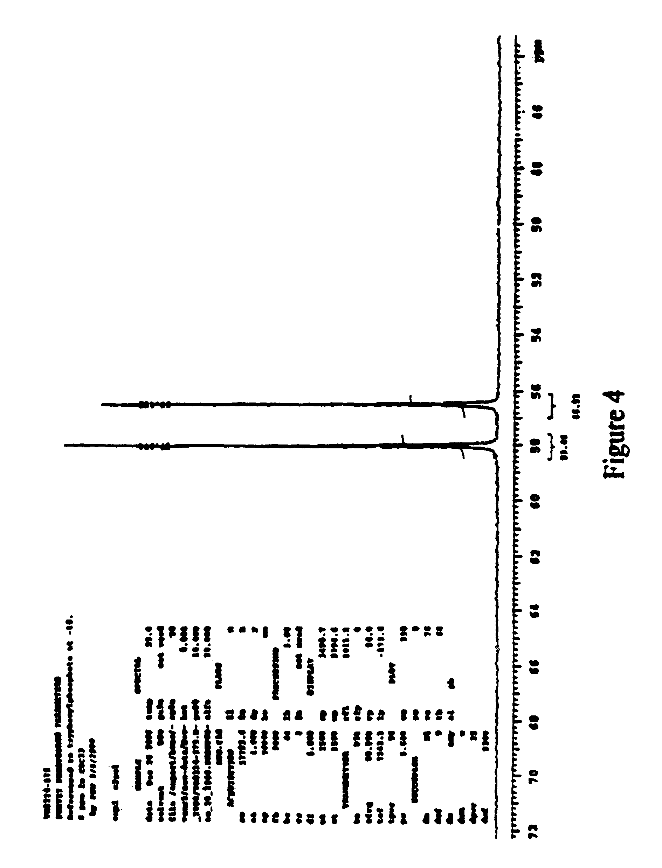

Table 1 clearly demonstrates that the presence of relatively bulky groups like 2'-*O*-methoxyethyl does not deter racemization and does not influence final chirality of the phosphorothioate linkage in a measurable manner. Thus 1*H*-tetrazole-catalyzed activation of phosphoramidites takes place with epimerization at the phosphorus center similar to the deoxyribonucleotide phosphoramidites. Consequently, initial enantiomeric excess present in the phosphoramidite monomer does not influence the ratio of the formed isomers. This conclusion was further substantiated by the following data (Table **2**) where phosphorothioate dimers were synthesized using 2'-*O*-methoxyethylguanosine phosphoramidite and analyzed by ³¹P NMR. A typical example of a ³¹P NMR of a monophosphorothioate linkage in a 1:1 enantiomeric ratio is shown in Figure 4. An important point to be observed in Table 1 is the reproducibility of the enantiomeric composition of the phosphorothioate linkage of the same oligonucleotide synthesized multiple times.

Tables **1** and **2** clearly show that it is unnecessary to enrich phosphoramidite composition to influence stereochemical outcome. Accordingly, commercially available phosphoramidites were used as received for further experiments.

**Table 2. Analysis of dimers using enantiomerically enriched 2'-O-MOE G amidite**

| **Expt #** | **Oligomer** | **Scale (µmole)** | **³¹P NMR (D₂O)** | |
|---|---|---|---|---|
| | | | **ppm (Rp/Sp)** | **Enantiomer Ratio** |
| 0225-150 | 5'-MOE G ps MOE U^{me}-3' | 29 | 57.94 / 56.24 | 47.73 / 52.27 |
| 0225-151 | 5'-MOE G ps MOE U^{me}-3' | 23 | 58.01 / 56.27 | 47.00 / 53.00 |
| 0225-152 | 5'-MOE G ps MOE U^{me}-3' | 30 | 58.05 / 56.32 | 47.04 / 52.96 |
| 0225-153 | 5'-MOE G ps MOE U^{me}-3' | 29 | 58.05 / 56.31 | 46.30 / 53.70 |
| | | | | |
| 0225-154 | 5'-MOE G ps T-3' | 32 | 57.77 / 56.53 | 45.15 / 54.85 |
| 0225-155 | 5'-MOE G ps T-3' | 32 | 57.76 / 56.54 | 45.75 / 54.25 |
| 0225-156 | 5'-MOE G ps T-3' | 33 | 57.76 / 56.54 | 45.53 / 54.47 |
| 0225-157 | 5'-MOE G ps T-3' | 34 | 57.76 / 56.54 | 45.90 / 54.10 |
| | | | | |
| 0224-140 | 5'- MOE G ps MOE A-3' | 188 | 57.62 / 56.50 | 42.01 / 57.99 |
| 0225-148 | 5'-MOE G ps MOE C^{me}-3' | 32 | 58.87 / 55.89 | 49.99 / 50.01 |
| 0225-159 | 5'-MOE G ps dG-3' | 30 | 57.41 / 56.76 | 51.60 / 48.40 |
| 0225-158 | 5'-MOE G ps dA-3' | 31 | 57.57 / 56.57 | 46.61 / 53.39 |
| 0225-160 | 5'-MOE G ps dC-3' | 25 | 57.84 / 56.45 | 47.60 / 52.40 |

Tables 1 and **2** clearly demonstrate that the stereochemistry of the phosphorothioate linkage is under complete control as indicated by the reproducible results of the ratio when a given oligomer is synthesized several times. It should be kept in mind that it is not the absolute numbers that matter but the relative ratios between multiple syntheses and between different bases.

Tables **3, 4** and **5** also reveal that coupling of deoxynucleotide phosphoramidites to 5'-hydroxyl of 2'-*O*-methoxyethylribonucleosides is also a racemic process leading to a 1:1 mixture of phosphorothioate linkages. Thus the coupling of 2'-*O*-MOE to deoxy, deoxy to 2'-*O*-MOE and 2'-*O*-MOE to 2'-*O*-MOE all lead to inherent net sterochemical control. It has been demonstrated that coupling of deoxy phosphoramidites to 5'-hydroxyl of deoxynucleoside/tide followed by sulfurization using phenylacetyl disulfide leads to stereocontrolled phosphorothioate linkages. Cheruvallath et al., Nucleosides, Nucleotides Nucleic Acids, 2000, 19, 533-543.

**Table 3. Analysis of 5-mers using various phosphoramidites**

| **Expt #** | **Oligomer** | **Scale (µmole)** | **³¹P NMR (D₂O)** | |
|---|---|---|---|---|
| | | | **ppm (Rp/Sp)** | **Enantiomer Ratio** |
| 0224-168 | 5'-MOE A ps TTTT-3' | 164 | 57.57 / 56.34 | 53.75 / 46.25 |
| 0224-169 | 5'-MOE A ps TTTT-3' | 163 | 57.60 / 56.28 | 53.96 / 46.04 |
| 0224-170 | 5'-MOE A ps TTTT-3' | 157 | 57.57 / 56.30 | 53.41 / 46.59 |
| 0224-171 | 5'-MOE A ps TTTT-3' | 157 | 57.58 / 56.33 | 53.79 / 46.21 |
| | | | | |
| 0224-172 | 5'-MOE A ps MOE A po TTT-3' | 160 | 57.99 / 56.46 | 53.45 / 46.55 |
| 0224-173 | 5'-MOE A ps MOE A po TTT-3' | 176 | 58.01 / 56.46 | 52.91 / 47.09 |
| 0224-174 | 5'-MOE A ps MOE A po TTT-3' | 171 | 57.97 / 56.47 | 52.95 / 47.05 |
| 0224-175 | 5'- MOE A ps MOE A po TTT-3' | 159 | 57.98 / 56.46 | 53.48 / 46.52 |
| | | | | |
| 0224-139 | 5'-MOE U^{me} ps TTTT-3' | 167 | 57.56 / 56.51 | 57.68 / 42.32 |
| 0224-135 | 5'-MOE U^{me} ps TTTT-3' | 176 | 57.57 / 56.54 | 57.82 / 42.18 |
| 0224-189 | 5'- MOE U^{me} ps TTTT-3' | 168 | 57.56 / 56.59 | 57.41 / 42.59 |
| 0224-190 | 5'-MOE U^{me} ps TTTT-3' | 164 | 57.58 / 56.60 | 57.47 / 42.53 |
| | | | | |
| 0224-136 | 5'-MOE U^{me}psMOE U^{me} poTTT-3' | 166 | 57.89 / 56.24 | 56.08 / 43.92 |
| 0750-1 | 5'-MOE U^{me}psMOE U^{me} poTTT-3' | 163 | 57.95 / 56.30 | 56.78 / 43.22 |
| 0750-2 | 5'- MOE U^{me}psMOE U^{me} poTTT-3' | 166 | 57.99 / 56.31 | 56.20 / 43.80 |
| 0750-3 | 5'- MOE U^{me}psMOE U^{me} poTTT-3' | 154 | 57.99 / 56.30 | 56.52 / 43.48 |
| | | | | |
| 0750-70 | 5'-dA ps MOE U^{me} po TTT-3' | 159 | 56.80 / 56.01 | 57.18 / 42.82 |
| 0750-71 | 5'-dA ps MOE U^{me} po TTT-3' | 167 | 56.80 / 55.99 | 57.05 / 42.95 |
| 0750-72 | 5'-dA ps MOE U^{me} poTTT-3' | 161 | 56.76 / 55.98 | 57.85 / 42.15 |
| 0750-73 | | 164 | | 57.79 / 42.21 |
| | 5'-dA ps MOE U^{me} po TTT-3' | | 56.80 / 55.99 | |
| 0750-74 | 5'-dG ps MOE U^{me} po TTT-3' | 169 | 56.64 / 56.07 | 47.57 / 52.43 |
| 0750-75 | 5'-dG ps MOE U^{me} po TTT-3' | 152 | 56.68 / 56.09 | 47.14 / 52.86 |
| 0750-76 | 5'-dG ps MOE U^{me} po TTT-3' | 157 | 56.65 / 56.07 | 47.19 / 52.81 |
| 0750-77 | 5'-dG ps MOE U^{me} po TTT-3' | 169 | 56.63 / 56.07 | 47.50 / 52.50 |
| | | | | |
| 0750-80 | 5'-dC ps MOE U^{me} po TTT-3' | 151 | 56.70 / 56.06 | 54.33 / 45.67 |
| 0750-81 | 5'-dC ps MOE U^{me} po TTT-3' | 155 | 56.76 / 56.09 | 54.81 / 45.19 |
| 0750-82 | 5'-dC ps MOE U^{me} po TTT-3' | 165 | 56.78 / 56.10 | 54.39 / 45.61 |
| 0750-83 | 5'-dC ps MOE U^{me} po TTT-3' | 156 | 56.76 / 56.09 | 54.84 / 45.16 |

It is well known that different synthesizers work differently viz., the bench top ABI 390Z DNA/RNA synthesizer uses an argon gas sparged reactor whereas the Amersham Pharmacia Biotech OligoPilot I, II and Akta synthesizers use a packed bed stainless steel column with no room for agitation. OligoProcess, a large-scale version of OligoPilot, is the only true large-scale synthesizer available. It is typically used for the routine manufacture of antisense drugs such as Vitravene™ as well for clinical trial evaluations. To determine whether a certain synthesizer contributes to any influence in stereoselectivity, oligomers synthesized on different synthesizers were evaluated. Additionally, whether the stereoselectivity of the phosphorothioate linkage is influenced by scale was determined. Tables 6, 7, and 8 present data of dimers obtained from the OligoPilot I, OligoPilot II, and Akta OligoPilot, respectively, and demonstrate that neither scale nor synthesizers influence the stereochemical outcome of the phosphorothioate linkage.

**Table 6. Analysis of dimers synthesized on OligoPilot I.**

| **Expt #** | **Dimer** | **Scale (µmole)** | **³¹P NMR (D₂O)** | |
|---|---|---|---|---|
| | | | **ppm (Rp/Sp)** | **Enantiomer Ratio** |
| 0225-152 | 5'-MOE G ps MOE U^{me} - 3' | 30 | 58.05 / 56.32 | 47.04 / 52.96 |
| 0225-159 | 5'-MOE G ps dG - 3' | 30 | 57.41 / 56.76 | 51.60 / 49.40 |
| 0225-148 | 5'-MOE G ps MOE C^{me} - 3' | 32 | 58.87 / 55.89 | 49.99 / 50.01 |
| 0225-167 | 5'-dC ps MOE U^{me} - 3' | 30 | 57.84 / 56.45 | 55.82 / 44.18 |

**Table 7. Analysis of dimers synthesized on OligoPilot II.**

| **Expt #** | **Dimer** | **Scale (µmole)** | **³¹P NMR (D₂O)** | |
|---|---|---|---|---|
| | | | **ppm (Rp/Sp)** | **Enantiomer Ratio** |
| 0224-82 | 5'-MOE G ps MOE U^{me} - 3' | 274 | 57.55 / 56.27 | 47.24 / 52.76 |
| 0224-142 | 5'-MOE G ps dG - 3' | 156 | 57.44 / 56.78 | 51.60 / 48.40 |
| 0224-84 | 5'-MOE G ps MOE C^{me} - 3' | 328 | 58.73 / 55.44 | 49.14 / 50.86 |
| 0750-146 | 5'-dC ps MOE U^{me} - 3' | 144 | 56.80 / 56.17 | 56.04 / 43.96 |

**Table 8. Analysis of dimers synthesized on Akta OligoPilot.**

| **Expt #** | **Dimer** | **Scale (µmole)** | **³¹P NMR (D₂O)** | |
|---|---|---|---|---|
| | | | **ppm (Rp/Sp)** | **Enantiomer Ratio** |
| 0750-190 | 5' -MOE G ps MOE U^{me} - 3' | 1000 | 57.95 / 56.32 | 48.07 / 51.93 |
| 0750-189 | 5'-MOE G ps dG - 3' | 1000 | 57.39 / 56.77 | 51.53 / 48.47 |
| 0750-188 | 5'-MOE G ps MOE C^{me} - 3' | 1000 | 58.96 / 55.74 | 49.24 / 50.76 |
| 0750-191 | 5'-dC ps MOE U^{me} - 3' | 1000 | 56.75 / 56.17 | 55.37 / 44.63 |

To determine whether the solid support used in oligonucleotide synthesis influences the stereochemistry of the phosphorothioate, oligonucleotides synthesized with different solid supports were studied. The data presented in Table 9 below indicates that the solid support does not influence stereochemistry.

**Table 9. Analysis of 5-mers using different supports synthesized on OligoPilot II.**

| **Expt #** | **Oligomer** | **Support** | **³¹P NMR (D₂O)** | |
|---|---|---|---|---|
| | | | **ppm (Rp/Sp)** | **Enantiomer Ratio** |
| | | | | |
| 0224-139 | 5'- MOE U^{me} ps TTTT-3' | HL 30 | 57.56 / 56.51 | 7.68 / 42.32 |
| 0750-149 | 5'- MOE U^{me} ps TTTT-3' | PS 200 | 57.62 / 56.55 | 7.52 / 42.48 |
| 0750-150 | 5'- MOE U^{me} ps TTTT-3' | Reloaded PS 200 | 57.62 / 56.54 | 6.86 / 43.14 |
| | | | | |
| 0224-136 | 5'-MOE U^{me}psMOE U^{me} TTT-3' | HL 30 | 57.89 / 56.24 | 6.08 / 43.92 |
| 0750-151 | 5'-MOE U^{me}psMOE U^{me} TTT-3' | PS 200 | 57.99 / 56.23 | 5.81 / 44.19 |
| 0750-152 | 5'-MOE U^{me}psMOE U^{me} TTT-3' | Reloaded PS 200 | 57.99 / 56.23 | 5.40 / 44.60 |
| | | | | |
| 0750-80 | 5'-dC ps MOE U^{me} po TTT-3' | HL 30 | 56.70 / 56.06 | 4.33 / 45.6 |
| 0750-147 | 5'-dC ps MOE U^{me} po TTT-3' | PS 200 | 56.78 / 56.07 | 4.38 / 45.62 |
| 0750-148 | 5'-dC ps MOE U^{me} po TTT-3' | Reloaded PS 200 | 56.78 / 56.07 | 4.23 / 45.77 |

The foregoing systematic evaluation of the various plausible contributors to the stereochemistry reveals that racemization is a rapid process, and that none of the factors identified above contributes in a measurable way to the net stereochemical outcome of the phosphorothioate linkage formation.

According to the present invention, it has been found that the pKa of the coupling agent used in the synthesis of oligonucleotides influences the stereochemical outcome of the phosphorothioate linkage. A coupling agent having a pKa ranging from about 3.3 to about 4.5, when used to couple a 2'-substituted nucleoside to a synthon comprising a nucleoside or a growing nucleotide chain results in an internucleotide phosphorothioate linkage that is enriched in the Rp enantiomer. A pKa below about 3.3 would cause removal of the protecting group at the 5'-hydroxyl position of the incoming 2'-substituted nucleoside. A pKa above about 4.5 does not enhance the ratio to any measurable extent until a pKa of about 6.5 is reached, which results in an internucleotide phosphorothioate linkage that is enriched in the Sp enantiomer. A coupling agent having a pKa from about 6.0 to 7.5 results in an internucleotide phosphorothioate linkage that is enriched in the Sp enantiomer. Table 10, below presents data comparing the coupling agents, 5-(ethylthio)-1*H*-tetrazole (ETT), 1H-tetrazole (1H-T), pyridium trifluoroacetate (PTFA), 4,5-dicyanoimidazole (DCI), and imidazolium triflate (ImTf). The data indicate that a pKa ranging from about 4.8 to about 5.5 does not significantly influence the stereochemical outcome of the phosphorothioate linkage.

**Table 10. Analysis of monophosphorothioate oligomers using different coupling agents-**

| **Expt #** | **Oligomer** | **Coupling Agent** | **pKa** | **³¹P NMR (D₂O)** | |
|---|---|---|---|---|---|
| | | | | **ppm (Rp/Sp)** | **Enantiomer Ratio** |
| 0762-62 | 5'- MOE U^{me} ps TTTT-3' | ETT | 4.3 | 57.54 / 56.52 | 69.61 / 30.39 |
| 0224-139 | 5'- MOE U^{me} ps TTTT-3' | 1H-T | 4.8 | 57.56 / 56.51 | 57.68 / 42.32 |
| 0750-156 | 5'- MOE U^{me} ps TTTT-3' | PTFA | ? | 57.51 / 56.52 | 51.19 / 48.81 |
| 0750-153 | 5'- MOE U^{me} ps TTTT-3' | DCI | 5.5 | 57.54 / 56.53 | 45.16 / 54.84 |
| 0762-112 | 5'- MOE U^{me} ps TTTT-3' | ImTf | 6.9 | 57.52 / 56.58 | 27.51 / 72.49 |
| | | | | | |
| 0762-63 | 5'-MOE U^{me}psMOE U^{me} TTT-3' | ETT | 4.3 | 4.3 | 69.00 / 31.00 |
| 0224-136 | 5'-MOE U^{me}psMOE U^{me} TTT-3' | 1H-T | 4.8 | 4.8 | 56.08 / 43.92 |
| 0750-157 | 5'-MOE U^{me}psMOE U^{me} TTT-3' | PTFA | ? | ? | 49.07 / 50.93 |
| 0750-154 | 5'-MOE U^{me}psMOE U^{me} TTT-3' | DCI | 5.5 | 5.5 | 45.44 / 54.56 |
| 0762-113 | 5'-MOE U^{me}psMOE U^{me} TTT-3' | ImTf | 6.9 | 6.9 | 25.82 / 74.18 |
| | | | | | |
| 0750-80 | 5'-dC ps MOE U^{me} po TTT-3' | 1H-T | 4.8 | 56.70 / 56.06 | 54.33 / 45.67 |
| 0750-158 | 5'-dC ps MOE U^{me} po TTT-3' | PTFA | ? | 56.60 / 56.03 | 55.28 / 44.72 |
| 0750-155 | 5'-dC ps MOE U^{me} po TTT-3' | DCI | 5.5 | 56.64 / 56.00 | 54.51 / 45.49 |
| | | | | | |
| 0750-70 | 5'-dA ps MOE U^{me} po TTT-3' | 1H-T | 4.8 | 56.80 / 56.01 | 57.18 / 42.82 |
| 0762-2 | 5'-dA ps MOE U^{me} po TTT-3' | PTFA | ? | 56.72 / 55.98 | 56.59 / 43.41 |
| 0762-3 | 5'-dA ps MOE U^{me} po TTT-3' | DCI | 5.5 | 56.72 / 55.98 | 57.49 / 42.51 |
| | | | | | |
| 0762-65 | 5'- MOE A ps MOE A po TTT-3' | ETT | 4.3 | 57.94 / 56.42 | 63.37 / 36.63 |
| 0224-172 | 5'- MOE A ps MOE A po TTT-3' | 1H-T | 4.8 | 57.99 / 56.46 | 53.45 / 46.55 |
| 0762-4 | 5'- MOE A ps MOE A po TTT-3' | PTFA | ? | 57.94 / 56.44 | 50.60 / 49.40 |
| 0762-5 | 5'- MOE A ps MOE A po TTT-3' | DCI | 5.5 | 57.79 / 56.46 | 45.62 / 54.38 |
| 0762-117 | 5'- MOE A ps MOE A po TTT-3' | ImTf | 6.9 | 57.86 / 56.46 | 33.41 / 66.59 |
| | | | | | |
| 0762-64 | 5'- MOE A ps TTTT-3' | ETT | 4.3 | 57.51 / 56.32 | 61.42 / 38.58 |
| 0224-168 | 5'- MOE A ps TTTT-3' | 1H-T | 4.8 | 57.57 / 56.34 | 53.75 / 46.25 |
| 0762-6 | 5'- MOE A ps TTTT-3' | PTFA | ? | 57.50 / 56.35 | 49.32 / 50.68 |
| 0762-1 | 5'- MOE A ps TTTT-3' | DCI | 5.5 | 57.44 / 56.38 | 41.32 / 58.68 |
| 0762-116 | 5'- MOE A ps TTTT-3' | ImTf | 6.9 | 57.53 / 56.32 | 32.27 / 67.73 |
| | | | | | |
| 0762-66 | 5'-MOE C^{me}ps TTTT-3' | ETT | 4.3 | 57.80 / 56.34 | 65.21 / 34.79 |
| 0762-72 | 5'-MOE C^{me}ps TTTT-3' | 1H-T | 4.8 | 57.88 / 56.36 | 52.97 / 47.03 |
| 0762-70 | 5'-MOE C^{me}ps TTTT-3' | PTFA | ? | 57.83 / 56.35 | 49.52 / 50.48 |
| 0762-68 | 5'-MOE C^{me}ps TTTT-3' | DCI | 5.5 | 57.82 / 56.34 | 41.86 / 58.14 |
| 0762-108 | 5'-MOE C^{me}ps TTTT-3' | ImTf | 6.9 | 57.82 / 56.39 | 30.31 / 69.69 |
| | | | | | |
| 0762-67 | 5'-MOE C^{me}ps MOE C^{me}poTTT-3' | ETT | 4.3 | 59.09 / 55.81 | 69.82 / 30.18 |
| 0762-73 | 5'-MOE C^{me}ps MOE C^{me}poTTT-3' | 1H-T | 4.8 | 58.94 / 55.81 | 55.30 / 44.70 |
| 0762-71 | 5'-MOE C^{me}ps MOE C^{me}poTTT-3' | PTFA | ? | 59.01 / 55.81 | 49.57 / 50.43 |
| 0762-69 | 5'-MOE C^{me}ps MOE C^{me}poTfT-3' | DCI | 5.5 | 58.87 / 55.80 | 41.72 / 58.28 |
| 0762-109 | 5'-MOE C^{me}ps MOE C^{me}poTTT-3' | ImTf | 6.9 | 58.94 / 55.85 | 30.12 / 69.88 |

The data from Table 10 indicates that coupling agents have much greater influence on the stereochemical ratio of phosphorothioate linkages than any other variable. It appears from the above table that the 2'-substituent of the nucleoside on the 5' end of the growing chain has very little influence on the outcome of the stereochemical ratio of the phosphorothioate linkage; that the 2'-substituents group of the incoming phosphoramidite greatly influences the stereochemical ratio; and that the size and/or pKa of the coupling agent is playing a significant role towards the enantiomeric ratio.

The present invention provides for the synthesis of oligonucleotides comprising regions that are connected by linkages of specific Rp or Sp chirality. For example, the 3' end of such an oligonucleotide may contain Sp linkages while the remainder of the oligonucleotide comprises Rp or achiral linkage. Any combination of linkages is possible using the methods of the present invention.

According to one embodiment, methods are provided for preparing oligonucleotides having defined regions of Sp and Rp linkages. For example, an oligonucleotide that is enriched with Sp linkages at the 3' end is prepared by employing a coupling agent having a pKa ranging from 6.0 to 7.5 at the beginning of the synthesis. If desired, the coupling agent is changed during synthesis to direct the synthesis of Rp linkages by employing a coupling agent having a pKa ranging from 3.3 to 4.5. It is possible to change the coupling agent at various points in the synthesis to provide, for example, oligonucleotides having one or more regions that are enhanced in the Sp enantiomer and/or one or more regions that are enhanced in the Rp enantiomer. The methods of the present invention are directed to preparing oligonucleotides with varying linkages. For example, oligonucleotides having one region of linkages that are not necessarily chirally pure may be flanked on either side by regions comprising phosphorothioate linkages that are enhanced in either the Rp or Sp enantiomer. Alternatively, it is possible to prepare oligonucleotides having an Rp region that is flanked by two regions on either side comprising regions that are enhanced in the Sp enantiomer.

The art skilled will easily recognize the various oligonucleotides that are produced using the methods of the present invention. Accordingly, the scope of the present claims is not limited to the examples set forth herein.

Oligonucleotides prepared by the methods of the present invention are expected to exhibit one or more efficacious properties such as, for example, hybridization with targeted RNA's and DNA's, cellular absorption and transport, or improved enzymatic interaction. At the same time, it is expected that these improvements to the basic oligonucleotide sequences will not significantly diminish existing properties of the basic oligonucleotide sequence. Thus, the present improvements are likely to lead to improved drugs, diagnostics, and research reagents.

As will be recognized additional objects, advantages, and novel features of this invention will become apparent to those skilled in the art upon examination of the following examples thereof, which are not intended to be limiting.

### EXPERIMENTAL

### Methods and Materials

Standard phosphoramidite monomer synthons, 5'-DMT-3'-*O*-(2-cyanoethyl)-*N,N*-diisopropyl phosphoramidites of thymidine, *N*⁴-benzoyl deoxycytidine, *N*²-isobutyryldeoxyguanosine, *N*⁶-benzoyldeoxyadenosine, 5-methyl-2'-*O-*methoxyethyluridine, 5-methyl-2'-*O*-methoxyethylcytidine, *N*⁶-benzoyl-2'-*O-*methoxyethyladenosine and *N*²-isobutyryl-2'-*O*-methoxyethylguanosine were purchased from Amersham-Pharmacia Biotech (Piscataway, NJ). 1*H*-Tetrazole was purchased from American International Company (Boston, MA). *tert*-Butyl hydroperoxide was purchased from Fluka Chemical Co. as a 70% aqueous solution. Phenylacetyl disulfide was purchased from H. C. Brown Labs (Mumbai, India). Anhydrous acetonitrile (<30 ppm water content) was purchased from Burdick & Jackson. Primer HL30 (loading ca 85 - 95 µmole/g) and PS 200 (loading ca 200 µmole/g) solid supports were purchased from Amersham-Pharmacia Biotech (Piscataway, NJ) (Table A). Reloaded PS 200 polystyrene solid support at a loading of 190 µmole/gram was prepared in-house using DMT thymidine succinate under a slightly modified condition. ³¹P NMR spectra were recorded on a Varian Unity Plus spectrometer at 161.9 MHz at room temperature. A minimum signal to noise ratio of 200 was obtained for all samples. Chemical shifts δ are given in ppm relative to H₃PO₄.

### Automated Synthesis of Monophosphorothioate Nucleotides

Oligonucleotide and dimer syntheses were performed on a Pharmacia OligoPilot I or II or Akta DNA/RNA synthesizer by the phosphoramidite method. The solid support was packed in a 1.6 or 6.3 ml stainless reactor column before use. Synthesis on the Akta DNA/RNA synthesizer was performed using a glass lined variable scale synthesis column. Typical synthesis scales on the OligoPilot I and OligoPilot II are in the ranges of 25-35 and 150-220 µmoles, respectively. Phosphate diester linkages were incorporated via oxidation of the phosphite triesters using a 15% (v/v) solution of *tert-*butyl hydroperoxide in acetonitrile at a flow rate of 5 ml / min for 15 min. Phosphorothioate linkages were introduced by sulfurization with 4 cm³ of a 0.2 M solution of phenylacetyl disulfide in acetonitrile/3-picoline (1:1 v/v) for a contact time of 2 min. Detritylation was effected by treatment with a 3% v/v solution of dichloroacetic acid in toluene for 4 min at a flow rate of 12.5 ml / min. Phosphoramidites were dissolved to a nominal concentration of 0.2 M in anhydrous acetonitrile and activated with two volumes of a 0.45 M solution of 1*H*-tetrazole in acetonitrile; couplings were performed in the recycle mode with a contact time of 5 min. Activation with pyridinium trifluoroacetate was carried out with a 0.22 M solution in acetonitrile in combination with 0.11 M solution of *N*-methylimidazole. Similarly, 4,5-dicyanoimidazole (DCI) was used as a 0.8 M solution in acetonitrile for activating phosphoramidites. Capping was performed using 4 ml of a 1:1 v/v mixture of acetic anhydride in acetonitrile (1:4 v/v) and *N*-methylimidazole-pyridine in acetonitrile (2:3:5 v/v/v) for a contact time of 1 min. Final detritylation at the end of synthesis was performed on the column before deprotection and cleavage.

### Deprotection and Analysis of Monophosphorothioate Nucleotides by ³¹P NMR Spectroscopy.

Following chain assembly the support-bound DMT-off oligonucleotide/dimer (300 mg) was treated with concentrated ammonium hydroxide (NH₄OH, 10 cm³) for 12 h at 55 °C. The products were filtered and the filtrate evaporated under reduced pressure. The residue was dissolved in deuterium oxide (1 ml) and carefully transferred to a 5 mm NMR tube for analysis.

### EXAMPLE I (Comparative)

### Synthesis of fully-modified 5'-[2'-O-methoxyethyl-(TTT-TTT-TTT-TTT-TTT-TTT-TT)-3' phosphorothioate 20-mer.

The synthesis of the above homo-pyrimidine sequence was performed on a Pharmacia OligoPilot II synthesizer on an 180 µmole scale using cyanoethyl phosphoramidite of 5'-O-DMT-2'-O-methoxyethyl-5-methyluridine. Pharmacia's HL 30 primer support loaded with 2'-O-methoxyethyl-5-methyluridine was used. Detritylation was performed using 3% dichloroacetic acid in toluene (volume/volume). Activation of phosphoramidite was done with a 0.45 M solution of 1H-tetrazole in acetonitrile. Sulfurization was performed using a 0.2 M solution of phenylacetyl disulfide in acetonitrile:3-picoline (1:1 v/v) for 2 minutes. At the end of synthesis, the support was treated with a solution of triethylamine:acetonitrile (1:1, v/v) for 12 hours, support washed with acetonitrile, oligo cleaved, and deprotected with 33% aqueous ammonium hydroxide at 55 °C for 12 hours, cooled, concentrated, and purified by reversed phase HPLC. All DMT fractions were combined and analyzed by capillary gel electrophoresis, detritylated, precipitated and lyophilized to a powder.

### EXAMPLE 2

### Synthesis of fully-modified 5'-[2'-O-methoxyethyl-(TTT-TTT-TTT-TTT-TTT-TTT-TT)-3' phosphorothioate 20-mer.

The synthesis of the above homo-pyrimidine sequence was performed on a Pharmacia OligoPilot II synthesizer on an 180 µmole scale using cyanoethyl phosphoramidite of 5'-O-DMT-2'-O-methoxyethyl-5-methyluridine. Pharmacia's HL 30 primer support loaded with 2'-O-methoxyethyl-5-methyluridine was used. Detritylation was performed using 3% dichloroacetic acid in toluene (volume/volume). Activation of phosphoramidite was done with a 0.22 M solution of pyridinium trifluoroacetate and 0.11 M solution of 1-methylimidazole in acetonitrile. Sulfurization was performed using a 0.2 M solution of phenylacetyl disulfide in acetonitrile:3-picoline (1:1 v/v) for 2 minutes. At the end of synthesis, the support was treated with a solution of triethylamine:acetonitrile (1:1, v/v) for 12 hours, support washed with acetonitrile, oligo cleaved, and deprotected with 33% aqueous ammonium hydroxide at 55°C for 12 hours, cooled, concentrated, and purified by reversed phase HPLC. All DMT fractions were combined and analyzed by capillary gel electrophoresis, detritylated, precipitated and lyophilized to a powder.

### EXAMPLE 3

### Synthesis of fully-modified 5'-[2'-O-methoxyethyl-(TT'f-TTT-TTT-TTT-TTT-TTT-TT)-3' phosphorothioate 20-mer.

The synthesis of the above homo-pyrimidine sequence was performed on a Pharmacia OligoPilot II synthesizer on a 180 µmole scale using cyanoethyl phosphoramidite of 5'-O-DMT-2'-O-methoxyethyl-5-methyluridine. Pharmacia's HL 30 primer support loaded with 2'-O-methoxyethyl-5-methyluridine was used. Detritylation was performed using 3% dichloroacetic acid in toluene (volume/volume). Activation of phosphoramidite was done with a 0.5 M solution of 4,5-dicyanoimidazole in acetonitrile. Sulfurization was performed using a 0.2 M solution of phenylacetyl disulfide in acetonitrile:3-picoline (1:1 v/v) for 2 minutes. At the end of synthesis, the support was treated with a solution of triethylamine:acetonitrile (1:1, v/v) for 12 hours, support washed with acetonitrile, oligo cleaved, and deprotected with 33% aqueous ammonium hydroxide at 55 °C for 12 hours, cooled, concentrated, and purified by reversed phase HPLC. All DMT fractions were combined and analyzed by capillary gel electrophoresis, detritylated, precipitated and lyophilized to a powder.

### EXAMPLE 4

### Synthesis of fully-modified 5'-[2'-O-methoxyethyl-(TTT-TTT-TTT-TTT-TTT-TTT-TT)-3' phosphorothioate 20-mer.

The synthesis of the above homo-pyrimidine sequence was performed on a Pharmacia OligoPilot, II synthesizer on a 180 µmole scale using cyanoethyl phosphoramidite of 5'-O-DMT-2'-O-methoxyethyl-5-methyluridine. Pharmacia's HL 30 primer support loaded with 2'-O-methoxyethyl-5-methyluridine was used. Detritylation was performed using 3% dichloroacetic acid in toluene (volume/volume). Activation of phosphoramidite was done with a 0.5 M solution of ethylthiotetrazole in acetonitrile. Sulfurization was performed using a 0.2 M solution of phenylacetyl disulfide in acetonitrile:3-picoline (1:1 v/v) for 2 minutes. At the end of synthesis, the support was treated with a solution of triethylamine:acetonitrile (1:1, v/v) for 12 hours, support washed with acetonitrile, oligo cleaved, and deprotected with 33% aqueous ammonium hydroxide at 55°C for 12 hours, cooled, concentrated, and purified by reversed phase HPLC. All DMT fractions were combined and analyzed by capillary gel electrophoresis, detritylated, precipitated and lyophilized to a powder.

### EXAMPLE 5 (Comparative)

### Synthesis of fully-modified 5'-[2'-O-methoxyethyl-(GCTGA]-d(TTA-GAG-AGA-G)-[2'-O-methoxyethyl-(GTCCQ-3' phosphorothioate 20-mer.

The synthesis of the above sequence was performed on a Pharmacia OligoPilot II synthesizer on a 180 µmole scale using cyanoethyl phosphoramidite of 2'-deoxyribonucleosides and 2'-O-methoxyethyl substituted ribonucleosides. Pharmacia's HL 30 primer support loaded with 2'-O-methoxyethyl-N4-benzoyl-5-methylcytidine was used. Detritylation was performed using 3% dichloroacetic acid in toluene (volume/volume). Activation of phosphoramidite was done with a 0.45 M solution of 1H-tetrazole in acetonitrile. Sulfurization was performed using a 0.2 M solution of phenylacetyl disulfide in acetonitrile:3-picoline (1:1 v/v) for 2 minutes. At the end of synthesis, the support was treated with a solution of triethylamine:acetonitrile (1:1, v/v) for 12 hours, support washed with acetonitrile, oligo cleaved, and deprotected with 33% aqueous ammonium hydroxide at 55 °C for 12 hours, cooled, concentrated, and purified by reversed phase HPLC. All DMT fractions were combined, detritylayed, precipitated and lyophilized to a powder. The stepwise Sulfurization efficiency was found to 99.7% based on ³¹P NMR (D₂O).

### EXAMPLE 6

### Synthesis of fully-modified 5'-[2'-O-methoxyethyl-(GCTGA)-d(TTA-GAG-AGA-G)-[2'-O-methoxyethyl-(GTCCC)-3' phosphorothioate 20-mer.

The synthesis of the above sequence was performed on a Pharmacia OligoPilot II synthesizer on a 180 µmole scale using cyanoethyl phosphoramidite of 2'-deoxyribonucleosides and 2'-O-methoxyethyl substituted ribonucleosides. Pharmacia's HL 30 primer support loaded with 2'-O-methoxyethyl-N4-benzoyl-5-methylcytidine was used. Detritylation was performed using 3% dichloroacetic acid in toluene (volume/volume). Activation of phosphoramidite was done with a 0.5 M solution of 4,5-dicyanoimidazole in acetonitrile. Sulfurization was performed using a 0.2 M solution of phenylacetyl disulfide in acetonitrile:3-picoline (1:1 v/v) for 2 minutes. At the end of synthesis, the support was treated with a solution of triethylamine:acetonitrile (1:1, v/v) for 12 hours, support washed with acetonitrile, oligo cleaved, and deprotected with 33% aqueous ammonium hydroxide at 55 °C for 12 hours, cooled, concentrated, and purified by reversed phase HPLC. All DMT fractions were combined, detritylayed, precipitated and lyophilized to a powder. The stepwise sulfurization efficiency was found to 99.7% based on ³¹P NMR (D₂O).

### EXAMPLE 7

### Synthesis of fully-modified 5'-[2'-O-methoxyethyl-(GCTGA]-d(TTA-GAG-AGA-G)-[2'-O-methoxyethyl-(GTCCC)-3' phosphorothioate 20-mer.

The synthesis of the above sequence was performed on a Pharmacia OligoPilot II synthesizer on a 180 µmole scale using cyanoethyl phosphoramidite of 2'-deoxyribonucleosides and 2'-O-methoxyethyl substituted ribonucleosides. Pharmacia's HL 30 primer support loaded with 2'-O-methoxyethyl-N4-benzoyl-5-methylcytidine was used. Detritylation was performed using 3% dichloroacetic acid in toluene (volume/volume). Activation of phosphoramidite was done with a 0.5 M solution of ethylthiotetrazole in acetonitrile. Sulfurization was performed using a 0.2 M solution of phenylacetyl disulfide in acetonitrile:3-picoline (1:1 v/v) for 2 minutes. At the end of synthesis, the support was treated with a solution of triethylamine:acetonitrile (1:1, v/v) for 12 hours, support washed with acetonitrile, oligo cleaved, and deprotected with 33% aqueous ammonium hydroxide at 55 °C for 12 hours, cooled, concentrated, and purified by reversed phase HPLC. All DMT fractions were combined, detritylayed, precipitated and lyophilized to a powder. The stepwise sulfurization efficiency was found to 99.7% based on ³¹P NMR (D₂O).

### EXAMPLE 8

### Synthesis of fully-modified 5'-[2'-O-methoxyethyl-(GCTGA]-d(TTA-GAG-AGA-G)-[2'-O-methoxyethyl-(GTCCC)-3' phosphorothioate 20-mer.

The synthesis of the above sequence was performed on a Pharmacia OligoPilot II synthesizer on a 180 µmole scale using cyanoethyl phosphoramidite of 2'-deoxyribonucleosides and 2'-O-methoxyethyl substituted ribonucleosides. Pharmacia's HL 30 primer support loaded with 2'-O-methoxyethyl-N4-benzoyl-5-methylcytidine was used. Detritylation was performed using 3% dichloroacetic acid in toluene (volume/volume). Activation of phosphoramidite was done with a 0.22 M solution of pyridinium trifluoroacetate and 0.11 M solution of 1-methylimidazole in acetonitrile. Sulfurization was performed using a 0.2 M solution of phenylacetyl disulfide in acetonitrile:3-picoline (1:1 v/v) for 2 minutes. At the end of synthesis, the support was treated with a solution of triethylamine:acetonitrile (1:1, v/v) for 12 hours, support washed with acetonitrile, oligo cleaved, and deprotected with 33% aqueous ammonium hydroxide at 55 °C for 12 hours, cooled, concentrated, and purified by reversed phase HPLC.. All DMT fractions were combined, detritylayed, precipitated and lyophilized to a powder. The stepwise sulfurization efficiency was found to 99.7% based on ³¹P NMR (D₂O).

### EXAMPLE 9 (Comparative)

### Synthesis of fully-modified 5'-[2'-O-methoxyethyl-(CTG]-d(AGT-CTG-TTT)-[2'-O-methoxyethyl-(TCC-ATT-CT)-3' phosphorothioate 20-mer.

The synthesis of the above sequence was performed on a Pharmacia OligoPilot II synthesizer on a 180 µmole scale using cyanoethyl phosphoramidite of 2'-deoxyribonucleosides and 2'-O-methoxyethyl substituted ribonucleosides. Pharmacia's HL 30 primer support loaded with 2'-O-methoxyethyl-5-methyluridine was used. Detritylation was performed using 3% dichloroacetic acid in toluene (volume/volume). Activation of phosphoramidite was done with a 0.45 M solution of 1H-tetrazole in acetonitrile. Sulfurization was performed using a 0.2 M solution of phenylacetyl disulfide in acetonitrile:3-picoline (1:1 v/v) for 2 minutes. At the end of synthesis, the support was treated with a solution of triethylamine:acetonitrile (1:1, v/v) for 12 hours, support washed with acetonitrile, oligo cleaved, and deprotected with 33% aqueous ammonium hydroxide at 55 °C for 12 hours, cooled, concentrated, and purified by reversed phase HPLC. All DMT fractions were combined, detritylated, precipitated and lyophilized to a powder. The stepwise sulfurization efficiency was found to 99.7% based on ³¹P NMR (D₂O).

### EXAMPLE 10

### Synthesis of fully-modified 5'-[2'-O-methoxyethyl-(CTG]-d(AGT-CTG-TTT)-[2'-O-methoxyethyl-(TCC-ATT-CT)-3' phosphorothioate 20-mer.

The synthesis of the above sequence was performed on a Pharmacia OligoPilot II synthesizer on a 180 µmole scale using cyanoethyl phosphoramidite of 2'-deoxyribonucleosides and 2'-O-methoxyethyl substituted ribonucleosides. Pharmacia's HL 30 primer support loaded with 2'-O-methoxyethyl-5-methyluridine was used. Detritylation was performed using 3% dichloroacetic acid in toluene (volume/volume). Activation of phosphoramidite was done with a 0.5 M solution of 4,5-dicyanoimidazole in acetonitrile. Sulfurization was performed using a 0.2 M solution of phenylacetyl disulfide in acetonitrile:3-picoline (1:1 v/v) for 2 minutes. At the end of synthesis, the support was treated with a solution of triethylamine:acetonitrile (1:1, v/v) for 12 hours, support washed with acetonitrile, oligo cleaved, and deprotected with 33% aqueous ammonium hydroxide at 55 °C for 12 hours, cooled, concentrated, and purified by reversed phase HPLC. All DMT fractions were combined, detritylated, precipitated and lyophilized to a powder. The stepwise sulfurization efficiency was found to 99.7% based on ³¹P NMR (D₂O).

### EXAMPLE 11

### Synthesis of fully-modified 5'-[2'-O-methoxyethyl-(CTG]-d(AGT-CTG-TTT)-[2'-O-methoxyethyl-(TCC-ATT-CT)-3' phosphorothioate 20-mer.

The synthesis of the above sequence was performed on a Pharmacia OligoPilot II synthesizer on a 180 µmole scale using cyanoethyl phosphoramidite of 2'-deoxyribonucleosides and 2'-O-methoxyethyl substituted ribonucleosides. Pharmacia's HL 30 primer support loaded with 2'-O-methoxyethyl-5-methyluridine was used. Detritylation was performed using 3% dichloroacetic acid in toluene (volume/volume). Activation of phosphoramidite was done with a 0.5 M solution of ethylthiotetrazole in acetonitrile. Sulfurization was performed using a 0.2 M solution of phenylacetyl disulfide in acetonitrile:3-picoline (1:1 v/v) for 2 minutes. At the end of synthesis, the support was treated with a solution of triethylamine:acetonitrile (1:1, v/v) for 12 hours, support washed with acetonitrile, oligo cleaved, and deprotected with 33% aqueous ammonium hydroxide at 55 °C for 12 hours, cooled, concentrated, and purified by reversed phase HPLC. All DMT fractions were combined, detritylated, precipitated and lyophilized to a powder. The stepwise sulfurization efficiency was found to 99.7% based on ³¹P NMR (D₂O).

### EXAMPLE 12

### Synthesis of fully-modified 5'-[2'-O-methoxyethyl-(CTG]-d(AGT-CTG-TTT)-[2'-O-methoxyethyl-(TCC-ATT-CT)-3' phosphorothioate 20-mer.

The synthesis of the above sequence was performed on a Pharmacia OligoPilot II synthesizer on a 180 µmole scale using cyanoethyl phosphoramidite of 2'-deoxyribonucleosides and 2'-O-methoxyethyl substituted ribonucleosides. Pharmacia's HL 30 primer support loaded with 2'-O-methoxyethyl-5-methyluridine was used. Detritylation was performed using 3% dichloroacetic acid in toluene (volume/volume). Activation of phosphoramidite was done with a 0.22 M solution of pyridinium trifluoroacetate and 0.11 M solution of 1-methylimidazole in acetonitrile. Sulfurization was performed using a 0.2 M solution of phenylacetyl disulfide in acetonitrile:3-picoline (1:1 v/v) for 2 minutes. At the end of synthesis, the support was treated with a solution of triethylamine:acetonitrile (1:1, v/v) for 12 hours, support washed with acetonitrile, oligo cleaved, and deprotected with 33% aqueous ammonium hydroxide at 55 °C for 12 hours, cooled, concentrated, and purified by reversed phase HPLC. All DMT fractions were combined, detritylated, precipitated and lyophilized to a powder. The stepwise sulfurization efficiency was found to 99.7% based on ³¹P NMR (D₂O).

### EXAMPLE 13 (Comparative)

### Synthesis of fully-modified 5'-[2'-O-methoxyethyl-(GTCTC]-d(GTT-GCG-TTT-G)-[2'-O-methoxyethyl-(TAG-TG)-3' phosphorothioate 20-mer.

The synthesis of the above sequence was performed on a Pharmacia OligoPilot II synthesizer on a 180 µmole scale using cyanoethyl phosphoramidite of 2'-deoxyribonucleosides and 2'-O-methoxyethyl substituted ribonucleosides. Pharmacia's HL 30 primer support loaded with 2'-O-methoxyethyl-5-methyluridine was used. Detritylation was performed using 3% dichloroacetic acid in toluene (volume/volume). Activation of phosphoramidite was done with a 0.45 M solution of 1H-tetrazole in acetonitrile. Sulfurization was performed using a 0.2 M solution of phenylacetyl disulfide in acetonitrile:3-picoline (1:1 v/v) for 2 minutes. At the end of synthesis, the support was treated with a solution of triethylamine:acetonitrile (1:1, v/v) for 12 hours, support washed with acetonitrile, oligo cleaved, and deprotected with 33% aqueous ammonium hydroxide at 55 °C for 12 hours, cooled, concentrated, and purified by reversed phase HPLC. All DMT fractions were combined, detritylated, precipitated and lyophilized to a powder. The stepwise sulfurization efficiency was found to 99.7% based on ³¹P NMR (D₂O).

### EXAMPLE 14

### Synthesis of fully-modified 5'-[2'-O-methoxyethyl-(GTCTC]-d(GTT-GCG-TTT-G)-[2'-O-methoxyethyl-(TAG-TG)-3' phosphorothioate 20-mer.

The synthesis of the above sequence was performed on a Pharmacia OligoPilot II synthesizer on a 180 µmole scale using cyanoethyl phosphoramidite of 2'-deoxyribonucleosides and 2'-O-methoxyethyl substituted ribonucleosides. Pharmacia's HL 30 primer support loaded with 2'-O-methoxyethyl-5-methyluridine was used. Detritylation was performed using 3% dichloroacetic acid in toluene (volume/volume). Activation of phosphoramidite was done with a 0.5 M solution of 4,5-dicyanoimidazole in acetonitrile. Sulfurization was performed using a 0.2 M solution of phenylacetyl disulfide in acetonitrile:3-picoline (1:1 v/v) for 2 minutes. At the end of synthesis, the support was treated with a solution of triethylamine:acetonitrile (1:1, v/v) for 12 hours, support washed with acetonitrile, oligo cleaved, and deprotected with 33% aqueous ammonium hydroxide at 55 °C for 12 hours, cooled, concentrated, and purified by reversed phase HPLC. All DMT fractions were combined, detritylated, precipitated and lyophilized to a powder. The stepwise sulfurization efficiency was found to 99.7% based on ³¹P NMR (D₂O).

### EXAMPLE 15

### Synthesis of fully-modified 5'-[2'-O-methoxyethyl-(GTCTC]-d(GTT-GCG-TTT-G)-[2'-O-methoxyethyl-(TAG-TG)-3' phosphorothioate 20-mer.

The synthesis of the above sequence was performed on a Pharmacia OligoPilot II synthesizer on a 180 µmole scale using cyanoethyl phosphoramidite of 2'-deoxyribonucleosides and 2'-O-methoxyethyl substituted ribonucleosides. Pharmacia's HL 30 primer support loaded with 2'-O-methoxyethyl-5-methyluridine was used. Detritylation was performed using 3% dichloroacetic acid in toluene (volume/volume). Activation of phosphoramidite was done with a 0.5 M solution of ethylthiotetrazole in acetonitrile. Sulfurization was performed using a 0.2 M solution of phenylacetyl disulfide in acetonitrile:3-picoline (1:1 v/v) for 2 minutes. At the end of synthesis, the support was treated with a solution of triethylamine:acetonitrile (1:1, v/v) for 12 hours, support washed with acetonitrile, oligo cleaved, and deprotected with 33% aqueous ammonium hydroxide at 55 °C for 12 hours, cooled, concentrated, and purified by reversed phase HPLC. All DMT fractions were combined, analyzed by capillary gel electrophoresis, detritylated, precipitated and lyophilized to a powder. The stepwise sulfurization efficiency was found to 99.7% based on ³¹P NMR (D₂O).

### EXAMPLE 16

### Synthesis of fully-modified 5'-[2'-O-methoxyethyl-(GTCTC]-d(GTT-GCG-TTT-G)-(2'-O-methoxyethyl-(TAG-TG)-3' phosphorothioate 20-mer.

The synthesis of the above sequence was performed on a Pharmacia OligoPilot II synthesizer on a 180 µmole scale using cyanoethyl phosphoramidite of 2'-deoxyribonucleosides and 2'-O-methoxyethyl substituted ribonucleosides. Pharmacia's HL 30 primer support loaded with 2'-O-methoxyethyl-5-methyluridine was used. Detritylation was performed using 3% dichloroacetic acid in toluene (volume/volume). Activation of phosphoramidite was done with a 0.22 M solution of pyridinium trifluoroacetate and 0.11 M solution of 1-methylimidazole in acetonitrile. Sulfurization was performed using a 0.2 M solution of phenylacetyl disulfide in acetonitrile:3-picoline (1:1 v/v) for 2 minutes. At the end of synthesis, the support was treated with a solution of triethylamine:acetonitrile (1:1, v/v) for 12 hours, support washed with acetonitrile, oligo cleaved, and deprotected with 33% aqueous ammonium hydroxide at 55 °C for 12 hours, cooled, concentrated, and purified by reversed phase HPLC. All DMT fractions were combined, detritylated, precipitated and lyophilized to a powder. The stepwise sulfurization efficiency was found to 99.7% based on ³¹P NMR (D₂O).

### EXAMPLE 17

### Synthesis of fully-modified 5'-[2'-O-methoxyethyl-(TCCCGC]-d(CTG-TGA-CA)-[2'-O-methoxyethyl-(TGC-ATT)-3' phosphorothioate 20-mer.

The synthesis of the above sequence was performed on a Pharmacia OligoPilot II synthesizer on a 180 µmole scale using cyanoethyl phosphoramidite of 2'-deoxyribonucleosides and 2'-O-methoxyethyl substituted ribonucleosides. Pharmacia's HL 30 primer support loaded with 2'-O-methoxyethyl-5-methyluridine was used. Detritylation was performed using 3% dichloroacetic acid in toluene (volume/volume). Activation of phosphoramidite was done with a 0.8 M solution of ethylthiotetrazole and 0.1 M solution of 1-methylimidazole in acetonitrile for the first wing of the gapmer from the 3' end. Activation of phosphoramidite was done with a 0.5 M solution of 4,5-dicyanoimidazole and 0.1 M solution of 1-methylimidazole in acetonitrile for the gap and second wing of the gapmer from the 3' end. Sulfurization was performed using a 0.2 M solution of phenylacetyl disulfide in acetonitrile:3-picoline (1:1 v/v) for 2 minutes. At the end of synthesis, the support was treated with a solution of triethylamine:acetonitrile (1:1, v/v) for 12 hours, support washed with acetonitrile, oligo cleaved, and deprotected with 33% aqueous ammonium hydroxide at 55 °C for 12 hours, cooled, concentrated, and purified by reversed phase HPLC. All DMT fractions were combined, detritylated, precipitated and lyophilized to a powder.

### EXAMPLE 18

### Synthesis of fully-modified 5'-[2'-O-methoxyethyl-(GCTCC]-d(TTC-CAC-TGAT)-[2'-O-methoxyethyl-(CCT-GC)-3' phosphorothioate 20-mer.

The synthesis of the above sequence was performed on a Pharmacia OligoPilot II synthesizer on a 180 µmole scale using cyanoethyl phosphoramidite of 2'-deoxyribonucleosides and 2'-O-methoxyethyl substituted ribonucleosides. Pharmacia's HL 30 primer support loaded with 2'-O-methoxyethyl-5-methyluridine was used. Detritylation was performed using 3% dichloroacetic acid in toluene (volume/volume). Activation of phosphoramidite was done with a 0.8 M solution of ethylthiotetrazole and 0.1 M solution of 1-methylimidazole in acetonitrile for the first wing of the gapmer from the 3' end. Activation of phosphoramidite was done with a 0.5 M solution of 4,5-dicyanoimidazole and 0.1 M solution of 1-methylimidazole in acetonitrile for the gap and second wing of the gapmer from the 3' end. Sulfurization was performed using a 0.2 M solution of phenylacetyl disulfide in acetonitrile:3-picoline (1:1 v/v) for 2 minutes. At the end of synthesis, the support was treated with a solution of triethylamine:acetonitrile (1:1, v/v) for 12 hours, support washed with acetonitrile, oligo cleaved, and deprotected with 33% aqueous ammonium hydroxide at 55 °C for 12 hours, cooled, concentrated, and purified by reversed phase HPLC. All DMT fractions were combined, detritylated, precipitated and lyophilized to a powder.

### EXAMPLE 19

### Synthesis of fully-modified 5'-[2'-O-methoxyethyl-(GCTGA]-d(TTA-GAG-AGA-G)-[2'-O-methoxyethyl-(GTCCC)-3' phosphorothioate 20-mer.

The synthesis of the above sequence was performed on a Pharmacia OligoPilot II synthesizer on a 180 µmole scale using cyanoethyl phosphoramidite of 2'-deoxyribonucleosides and 2'-O-methoxyethyl substituted ribonucleosides. Pharmacia's HL 30 primer support loaded with 2'-O-methoxyethyl-N4-benzoyl-5-methylcytidine was used. Detritylation was performed using 3% dichloroacetic acid in toluene (volume/volume). Activation of phosphoramidite was done with a 0.4 M solution of imidazolium triflate in acetonitrile. Sulfurization was performed using a 0.2 M solution of phenylacetyl disulfide in acetonitrile:3-picoline (1:1 v/v) for 2 minutes. At the end of synthesis, the support was treated with a solution of triethylamine:acetonitrile (1:1, v/v) for 12 hours, support washed with acetonitrile, oligo cleaved, and deprotected with 33% aqueous ammonium hydroxide at 55 °C for 12 hours, cooled, concentrated, and purified by reversed phase HPLC. All DMT fractions were combined, detritylayed, precipitated and lyophilized to a powder. The stepwise sulfurization efficiency was found to 99.7% based on ³¹P NMR (D₂O).

### EXAMPLE 20

### Synthesis of fully-modified 5'-[2'-O-methoxyethyl-(CTG]-d(AGT-CTG-TTT)-[2'-O-methoxyethyl-(TCC-ATT-CT)-3' phosphorothioate 20-mer.

The synthesis of the above sequence was performed on a Pharmacia OligoPilot II synthesizer on a 180 µmole scale using cyanoethyl phosphoramidite of 2'-deoxyribonucleosides and 2'-O-methoxyethyl substituted ribonucleosides. Pharmacia's HL 30 primer support loaded with 2'-O-methoxyethyl-5-methyluridine was used. Detritylation was performed using 3% dichloroacetic acid in toluene (volume/volume). Activation of phosphoramidite was done with a 0.4 M solution of imidazolium triflate in acetonitrile. Sulfurization was performed using a 0.2 M solution of phenylacetyl disulfide in acetonitrile:3-picoline (1:1 v/v) for 2 minutes. At the end of synthesis, the support was treated with a solution of triethylamine:acetonitrile (1:1, v/v) for 12 hours, support washed with acetonitrile, oligo cleaved, and deprotected with 33% aqueous ammonium hydroxide at 55 °C for 12 hours, cooled, concentrated, and purified by reversed phase HPLC. All DMT fractions were combined, detritylated, precipitated and lyophilized to a powder. The stepwise sulfurization efficiency was found to 99.7% based on ³¹P NMR (D₂O).

### EXAMPLE 21

### Synthesis of fully-modified 5'-[2'-O-methyl-(TTT-TTT-TTT-TTT-TTT-TTT-TT)-3' phosphorothioate 20-mer.

The synthesis of the above homo-pyrimidine sequence was performed on a Pharmacia OligoPilot II synthesizer on a 180 µmole scale using cyanoethyl phosphoramidite of 5'-O-DMT-2'-O-methyl-5-methyluridine. Pharmacia's HL 30 primer support loaded with 2'-O-methyl-5-methyluridine was used. Detritylation was performed using 3% dichloroacetic acid in toluene (volume/volume). Activation of phosphoramidite was done with a 0.4 M solution of ethylthiotetrazole in acetonitrile. Sulfurization was performed using a 0.2 M solution of phenylacetyl disulfide in acetonitrile:3-picoline (1:1 v/v) for 2 minutes. At the end of synthesis, the support was treated with a solution of triethylamine:acetonitrile (1:1, v/v) for 12 hours, support washed with acetonitrile, oligo cleaved, and deprotected with 33% aqueous ammonium hydroxide at 55 °C for 12 hours, cooled, concentrated, and purified by reversed phase HPLC. All DMT fractions were combined, analyzed by capillary gel electrophoresis, detritylated, precipitated and lyophilized to a powder.

### EXAMPLE 22

### Synthesis of fully-modified 5'-[2'-O-methyl-(TTT-TTT-TTT-TTT-TTT-TTT-TT)-3' phosphorothioate 20-mer.

The synthesis of the above homo-pyrimidine sequence was performed on a Pharmacia OligoPilot II synthesizer on a 180 µmole scale using cyanoethyl phosphoramidite of 5'-O-DMT-2'-O-methyl-5-methyluridine. Pharmacia's HL 30 primer support loaded with 2'-O-methyl-5-methyluridine was used. Detritylation was performed using 3% dichloroacetic acid in toluene (volume/volume). Activation of phosphoramidite was done with a 0.4 M solution of imidazolium triflate in acetonitrile. Sulfurization was performed using a 0.2 M solution of phenylacetyl disulfide in acetonitrile:3-picoline (1:1 v/v) for 2 minutes. At the end of synthesis, the support was treated with a solution of triethylamine:acetonitrile (1:1, v/v) for 12 hours, support washed with acetonitrile, oligo cleaved, and deprotected with 33% aqueous ammonium hydroxide at 55 °C for 12 hours, cooled, concentrated, and purified by reversed phase HPLC. All DMT fractions were combined, analyzed by capillary gel electrophoresis, detritylated, precipitated and lyophilized to a powder.

### EXAMPLE 23

### Synthesis of fully-moditied 5'-[2'-O-methyl-(TTT-TTT-TTT-TTT-TTT-TTT-TT)-3' phosphorothioate 20-mer.

The synthesis of the above homo-pyrimidine sequence was performed on a Pharmacia OligoPilot II synthesizer on a 180 µmole scale using cyanoethyl phosphoramidite of 5'-O-DMT-2'-O-methyl-5-methyluridine. Pharmacia's HL 30 primer support loaded with 2'-O-methyl-5-methyluridine was used. Detritylation was performed using 3% dichloroacetic acid in toluene (volume/volume). Activation of phosphoramidite was done with a 0.4 M solution of 4,5-dicyanoimidazole in acetonitrile. Sulfurization was performed using a 0.2 M solution of phenylacetyl disulfide in acetonitrile:3-picoline (1:1 v/v) for 2 minutes. At the end of synthesis, the support was treated with a solution of triethylamine:acetonitrile (1:1, v/v) for 12 hours, support washed with acetonitrile, oligo cleaved, and deprotected with 33% aqueous ammonium hydroxide at 55°C for 12 hours, cooled, concentrated, and purified by reversed phase HPLC. All DMT fractions were combined, analyzed by capillary gel electrophoresis, detritylated, precipitated and lyophilized to a powder.

### EXAMPLE 24

### Synthesis of fully-modified 5'-[2'-O-methyl-(GCTGA]-d(TTA-GAG-AGA-G)-[2'-O-methyl-(GTCCC)-3' phosphorothioate 20-mer.

The synthesis of the above sequence was performed on a Pharmacia OligoPilot II synthesizer on a 180 µmole scale using cyanoethyl phosphoramidite of 2'-deoxyribonucleosides and 2'-O-methyl substituted ribonucleosides. Pharmacia's HL 30 primer support loaded with 2'-O-methyl-N4-benzoyl-5-methylcytidine was used. Detritylation was performed using 3% dichloroacetic acid in toluene (volume/volume). Activation of phosphoramidite was done with a 0.22 M solution of pyridinium trifluoroacetate and 0.11 M solution of 1-methylimidazole in acetonitrile. Sulfurization was performed using a 0.2 M solution of phenylacetyl disulfide in acetonitrile:3-picoline (1:1 v/v) for 2 minutes. At the end of synthesis, the support was treated with a solution of triethylamine:acetonitrile (1:1, v/v) for 12 hours, support washed with acetonitrile, oligo cleaved, and deprotected with 33% aqueous ammonium hydroxide at 55 °C for 12 hours, cooled, concentrated, and purified by reversed phase HPLC. All DMT fractions were combined, detritylayed, precipitated and lyophilized to a powder. The stepwise sulfurization efficiency was found to 99.7% based on ³¹P NMR (D₂O).

### EXAMPLE 25

### Synthesis of fully-modified 5'-[2'-O-methyl-(CTG]-d(AGT-CTG-TTT)-[2'-O-methyl-(TCC-ATT-CT)-3' phosphorothioate 20-mer.

The synthesis of the above sequence was performed on a Pharmacia OligoPilot II synthesizer on a 180 µmole scale using cyanoethyl phosphoramidite of 2'-deoxyribonucleosides and 2'-O-methyl substituted ribonucleosides. Pharmacia's HL 30 primer support loaded with 2'-O-methyl-5-methyluridine was used. Detritylation was performed using 3% dichloroacetic acid in toluene (volume/volume). Activation of phosphoramidite was done with a 0.4 M solution of ethylthiotetrazole in acetonitrile. Sulfurization was performed using a 0.2 M solution of phenylacetyl disulfide in acetonitrile:3-picoline (1:1 v/v) for 2 minutes. At the end of synthesis, the support was treated with a solution of triethylamine:acetonitrile (1:1, v/v) for 12 hours, support washed with acetonitrile, oligo cleaved, and deprotected with 33% aqueous ammonium hydroxide at 55 °C for 12 hours, cooled, concentrated, and purified by reversed phase HPLC. All DMT fractions were combined, detritylated, precipitated and lyophilized to a powder. The stepwise sulfurization efficiency was found to 99.7% based on ³¹P NMR (D₂O).

### EXAMPLE 26

### Synthesis of fully-modified 5'-[2'-O-methyl-(CTG]-d(AGT-CTG-TTT)-[2'-O-methyl-(TCC-ATT-CT)-3' phosphorothioate 20-mer.

The synthesis of the above sequence was performed on a Pharmacia OligoPilot II synthesizer on a 180 µmole scale using cyanoethyl phosphoramidite of 2'-deoxyribonucleosides and 2'-O-methyl substituted ribonucleosides. Pharmacia's HL 30 primer support loaded with 2'-O-methyl-5-methyluridine was used. Detritylation was performed using 3% dichloroacetic acid in toluene (volume/volume). Activation of phosphoramidite was done with a 0.4 M solution of imidazolium triflate in acetonitrile. Sulfurization was performed using a 0.2 M solution of phenylacetyl disulfide in acetonitrile:3-picoline (1:1 v/v) for 2 minutes. At the end of synthesis, the support was treated with a solution of triethylamine:acetonitrile (1:1, v/v) for 12 hours, support washed with acetonitrile, oligo cleaved, and deprotected with 33% aqueous ammonium hydroxide at 55 °C for 12 hours, cooled, concentrated, and purified by reversed phase HPLC. All DMT fractions were combined, detritylated, precipitated and lyophilized to a powder. The stepwise sulfurization efficiency was found to 99.7% based on ³¹P NMR (D₂O).

### EXAMPLE 27

### Synthesis of fully-modified 5'-[2'-O-methyl-(CTG]-d(AGT-CTG-TTT)-[2'-O-methyl-(TCC-ATT-CT)-3' phosphorothioate 20-mer.

The synthesis of the above sequence was performed on a Pharmacia OligoPilot II synthesizer on a 180 µmole scale using cyanoethyl phosphoramidite of 2'-deoxyribonucleosides and 2'-O-methyl substituted ribonucleosides. Pharmacia's HL 30 primer support loaded with 2'-O-methyl-5-methyluridine was used. Detritylation was performed using 3% dichloroacetic acid in toluene (volume/volume). Activation of phosphoramidite was done with a 0.4 M solution of 4,5-dicyanoimidazole in acetonitrile. Sulfurization was performed using a 0.2 M solution of phenylacetyl disulfide in acetonitrile:3-picoline (1:1 v/v) for 2 minutes. At the end of synthesis, the support was treated with a solution of triethylamine:acetonitrile (1:1, v/v) for 12 hours, support washed with acetonitrile, oligo cleaved, and deprotected with 33% aqueous ammonium hydroxide at 55 °C for 12 hours, cooled, concentrated, and purified by reversed phase HPLC. All DMT fractions were combined, detritylated, precipitated and lyophilized to a powder. The stepwise sulfurization efficiency was found to 99.7% based on ³¹P NMR (D₂O).

### EXAMPLE 28

### Synthesis of fully-modified 5'-[2'-O-methoxyethyl-(TTT-TTT-TTT-TTT-TTT-TTT-TT)-3' phosphorothioate 20-mer.

The synthesis of the above homo-pyrimidine sequence was performed on a Pharmacia OligoPilot II synthesizer on a 180 µmole scale using 2-(2'-acetoxyphenoxy)ethyl phosphoramidite of 5'-O-DMT-2'-O-methoxyethyl-5-methyluridine. Pharmacia's HL 30 primer support loaded with 2'-O-methoxyethyl-5-methyluridine was used. Detritylation was performed using 3% dichloroacetic acid in toluene (volume/volume). Activation of phosphoramidite was done with a 0.4 M solution of ethylthiotetrazole in acetonitrile. Sulfurization was performed using a 0.2 M solution of phenylacetyl disulfide in acetonitrile:3-picoline (1:1 v/v) for 2 minutes. At the end of synthesis, the support was treated with a solution oftriethylamine:acetonitrile (1:1, v/v) for 12 hours, support washed with acetonitrile, oligo cleaved, and deprotected with 33% aqueous ammonium hydroxide at 55 °C for 12 hours, cooled, concentrated, and purified by reversed phase HPLC. All DMT fractions were combined, analyzed by capillary gel electrophoresis, detritylated, precipitated and lyophilized to a powder.

### EXAMPLE 29

### Synthesis of fully-modified 5'-[2'-O-methoxyethyl-(TTT-TTT-TTT-TTT-TTT-TTT-TT)-3' phosphorothioate 20-mer.

The synthesis of the above homo-pyrimidine sequence was performed on a Pharmacia OligoPilot II synthesizer on a 180 µmole scale using 2-(2'-acetoxyphenoxy)ethyl phosphoramidite of 5'-O-DMT-2'-O-methoxyethyl-5-methyluridine. Pharmacia's HL 30 primer support loaded with 2'-O-methoxyethyl-5-methyluridine was used. Detritylation was performed using 3% dichloroacetic acid in toluene (volume/volume). Activation of phosphoramidite was done with a 0.4 M solution of imidazolium triflate in acetonitrile. Sulfurization was performed using a 0.2 M solution of phenylacetyl disulfide in acetonitrile:3-picoline (1:1 v/v) for 2 minutes. At the end of synthesis, the support was treated with a solution of triethylamine:acetonitrile (1:1, v/v) for 12 hours, support washed with acetonitrile, oligo cleaved, and deprotected with 33% aqueous ammonium hydroxide at 55 °C for 12 hours, cooled, concentrated, and purified by reversed phase HPLC. All DMT fractions were combined, analyzed by capillary gel electrophoresis, detritylated, precipitated and lyophilized to a powder.

### EXAMPLE 30

### Synthesis of fully-modified 5'-[2'-O-methoxyethyl-(TTT-TTT-TTT-TTT-TTT-TTT-TT)-3' phosphorothioate 20-mer.

The synthesis of the above homo-pyrimidine sequence was performed on a Pharmacia OligoPilot II synthesizer on a 180 µmole scale using 2-(2'-acetoxyphenoxy)ethyl phosphoramidite of 5'-O-DMT-2'-O-methoxyethyl-5-methyluridine. Pharmacia's HL 30 primer support loaded with 2'-O-methoxyethyl-5-methyluridine was used. Detritylation was performed using 3% dichloroacetic acid in toluene (volume/volume). Activation of phosphoramidite was done with a 0.4 M solution of 4,5-dicyanoimidazole in acetonitrile. Sulfurization was performed using a 0.2 M solution of phenylacetyl disulfide in acetonitrile:3-picoline (1:1 v/v) for 2 minutes. At the end of synthesis, the support was treated with a solution of triethylamine:acetonitrile (1:1, v/v) for 12 hours, support washed with acetonitrile, oligo cleaved, and deprotected with 33% aqueous ammonium hydroxide at 55 °C for 12 hours, cooled, concentrated, and purified by reversed phase HPLC. All DMT fractions were combined, analyzed by capillary gel electrophoresis, detritylated, precipitated and lyophilized to a powder.

### EXAMPLE 31

### Synthesis of fully-modified 5'-[2'-O-methoxyethyl-(GCTGA]-d(TTA-GAG-AGA-G)-[2'-O-methoxyethyl-(GTCCC)-3' phosphorothioate 20-mer.

The synthesis of the above sequence was performed on a Pharmacia OligoPilot II synthesizer on a 180 µmole scale using 2-(2'-acetoxyphenoxy)ethyl phosphoramidite of 2'-deoxyribonucleosides and 2'-O-methoxyethyl substituted ribonucleosides. Pharmacia's HL 30 primer support loaded with 2'-O-methoxyethyl-N4-benzoyl-5-methylcytidine was used. Detritylation was performed using 3% dichloroacetic acid in toluene (volume/volume). Activation of phosphoramidite was done with a 0.22 M solution of pyridinium trifluoroacetate and 0.11 M solution of 1-methylimidazole in acetonitrile. Sulfurization was performed using a 0.2 M solution of phenylacetyl disulfide in acetonitrile:3-picoline (1:1 v/v) for 2 minutes. At the end of synthesis, the support was treated with a solution of triethylamine:acetonitrile (1:1, v/v) for 12 hours, support washed with acetonitrile, oligo cleaved, and deprotected with 33% aqueous ammonium hydroxide at 55 °C for 12 hours, cooled, concentrated, and purified by reversed phase HPLC. All DMT fractions were combined, detritylayed, precipitated and lyophilized to a powder. The stepwise sulfurization efficiency was found to 99.7% based on ³¹P NMR (D₂O).

### EXAMPLE 32

### Synthesis of fully-modified 5'-[2'-O-methoxyethyl-(CTG]-d(AGT-CTG-TTT)-[2'-O-methoxyethyl-(TCC-ATT-CT)-3' phosphorothioate 20-mer.

The synthesis of the above sequence was performed on a Pharmacia OligoPilot II synthesizer on a 172 µmole scale using 2-(2'-acetoxyphenoxy)ethyl phosphoramidite of 2'-deoxyribonucleosides and 2'-O-methoxyethyl substituted ribonucleosides. Pharmacia's HL 30 primer support loaded with 2'-O-methoxyethyl-5-methyluridine was used. Detritylation was performed using 3% dichloroacetic acid in toluene (volume/volume). Activation of phosphoramidite was done with a 0.4 M solution of ethylthiotetrazole in acetonitrile. Sulfurization was performed using a 0.2 M solution of phenylacetyl disulfide in acetonitrile:3-picoline (1:1 v/v) for 2 minutes. At the end of synthesis, the support was treated with a solution of triethylamine:acetonitrile (1:1, v/v) for 12 hours, support washed with acetonitrile, oligo cleaved, and deprotected with 33% aqueous ammonium hydroxide at 55 °C for 12 hours, cooled, concentrated, and purified by reversed phase HPLC. All DMT fractions were combined, detritylated, precipitated and lyophilized to a powder. The stepwise sulfurization efficiency was found to 99.7% based on ³¹P NMR (D₂O).

### EXAMPLE 33

### Synthesis of fully-modified 5'-[2'-O-methoxyethyl-(CTG]-d(AGT-CTG-TTT)-[2'-O-methoxyethyl-(TCC-ATT-CT)-3' phosphorothioate 20-mer.

The synthesis of the above sequence was performed on a Pharmacia OligoPilot II synthesizer on a 180 µmole scale using 2-(2'-acetoxyphenoxy)ethyl phosphoramidite of 2'-deoxyribonucleosides and 2'-O-methoxyethyl substituted ribonucleosides. Pharmacia's HL 30 primer support loaded with 2'-O-methoxyethyl-5-methyluridine was used. Detritylation was performed using 3% dichloroacetic acid in toluene (volume/volume). Activation of phosphoramidite was done with a 0.4 M solution of imidazolium triflate in acetonitrile. Sulfurization was performed using a 0.2 M solution of phenylacetyl disulfide in acetonitrile:3-picoline (1:1 v/v) for 2 minutes. At the end of synthesis, the support was treated with a solution of triethylamine:acetonitrile (1:1, v/v) for 12 hours, support washed with acetonitrile, oligo cleaved, and deprotected with 33% aqueous ammonium hydroxide at 55 °C for 12 hours, cooled, concentrated, and purified by reversed phase HPLC. All DMT fractions were combined, detritylated, precipitated and lyophilized to a powder. The stepwise sulfurization efficiency was found to 99.7% based on ³¹P NMR (D₂O).

### EXAMPLE 34

### Synthesis of fully-modified 5'-[2'-O-methoxyethyl-(CTG]-d(AGT-CTG-TTT)-[2'-O-methoxyethyl-(TCC-ATT-CT)-3' phosphorothioate 20-mer.

The synthesis of the above sequence was performed on a Pharmacia OligoPilot II synthesizer on a 180 µmole scale using 2-(2'-acetoxyphenoxy)ethyl phosphoramidite of 2'-deoxyribonucleosides and 2'-O-methoxyethyl substituted ribonucleosides. Pharmacia's HL 30 primer support loaded with 2'-O-methoxyethyl-5-methyluridine was used. Detritylation was performed using 3% dichloroacetic acid in toluene (volume/volume). Activation of phosphoramidite was done with a 0.4 M solution of 4,5-dicyanoimidazole in acetonitrile. Sulfurization was performed using a 0.2 M solution of phenylacetyl disulfide in acetonitrile:3-picoline (1:1 v/v) for 2 minutes. At the end of synthesis, the support was treated with a solution of triethylamine:acetonitrile (1:1, v/v) for 12 hours, support washed with acetonitrile, oligo cleaved, and deprotected with 33% aqueous ammonium hydroxide at 55 °C for 12 hours, cooled, concentrated, and purified by reversed phase HPLC. All DMT fractions were combined, detritylated, precipitated and lyophilized to a powder. The stepwise sulfurization efficiency was found to 99.7% based on ³¹P NMR (D₂O).

## Claims

1. A method for preparing an internucleotide phosphorothioate linkage enriched in the Rp enantiomer between a synthon having a hydroxyl moiety at the 5' position and a 2'-substituted nucleoside having an activated phosphate moiety at the 3'-position comprising:
selecting a coupling agent having a pKa ranging from 3.3 to 4.5;
coupling said synthon to said 2'-substituted nucleoside in the presence of said coupling agent to form a phosphite intermediate; and
oxidising said phosphite intermediate with phenylacetyl disulfide.

2. The method of claim 1 wherein said first synthon is bound to a support.

3. The method of claim 1 wherein the synthon having a hydroxyl moiety at the 5' position is a deoxyribonucleotide, a ribonucleotide, a 2'-protected ribonucleotide or a 2'-substitued ribonucleotide.

4. The method of claim 1 wherein said coupling agent has a pKa ranging from 3.4 to 4.4.

5. The method of claim 1 wherein said coupling agent has a pKa ranging from 3.5 to 4.3.

6. The method of claim 1 wherein said coupling agent has a pKa ranging from 3.6 to 4.3.

7. The method of claim 1 wherein said coupling agent has a pKa ranging from 3.7 to 4.3.

8. The method of claim 1 wherein said coupling agent is 5-(ethylthio)-1H-tetrazole.

9. The method of claim 1 wherein said 2'-substituent is attached to the 2'-position through an oxygen atom.

10. The method of claim 9 wherein said 2'-substituent is O-alkyl, O-PG, O(CH₂)ₙOCH₃, or O[(CH₂)ₙO]ₘCH₃, wherein PG is a hydroxyl protecting group and n and m are from 1 to 10.

11. The method of claim 10 wherein said 2'-substituent is 2'-O-alkyl.

12. The method of claim 11 wherein said alkyl group is C₁ to C₁₂ alkyl.

13. The method of claim 12 wherein said alkyl group is methyl.

14. The method of claim 10 wherein said 2'-substituent is O(CH₂)ₙOCH₃ and n is from 1 to 3.

15. The method of claim 9 wherein said 2'-substituent is O(CH₂)₂OCH₃.

16. The method of claim 1 wherein said activated phosphate moiety comprises a B-cyanoethyl protecting group.

17. The method of claim 1 wherein said activated phosphate moiety comprises an acetoxy phenoxy ethyl group.

18. A method for preparing an internucleotide phosphorothioate linkage enriched in the Sp enantiomer between a synthon having a hydroxyl moiety at the 5' position and a 2'-substituted nucleoside having an activated phosphate moiety at the 3'-position comprising:
selecting a coupling agent having a pKa ranging from 6.0 to 8.0;
coupling said synthon to said 2'- substituted nucleoside in the presence of said coupling agent; and measuring the enantiomeric ratio.

19. The method of claim 18 wherein said synthon is bound to a support.

20. The method of claim 18 wherein the synthon having a hydroxyl moiety at the 5' position is a deoxyribonucleotide, a ribonucleotide, a 2'-protected ribonucleotide or a 2'-substitued ribonucleotide.

21. The method of claim 18 wherein said coupling agent has a pKa ranging from 6.5 to 7.5.

22. The method of claim 18 wherein said coupling agent has a pKa ranging from 6.2 to 7.3.

23. The method of claim 18 wherein said coupling agent has a pKa ranging from 6.4 to 7.1.

24. The method of claim 18 wherein said coupling agent has a pKa ranging from 6.5 to 7.0.

25. The method of claim 18 wherein said coupling agent has a pKa ranging from 6.7 to 6.9.

26. The method of claim 18 wherein said coupling agent is an imidazolium or pyridinium derivative.

27. The method of claim 26 wherein said coupling agent is an imidazolium or pyridinium salt.

28. The method of claim 27 wherein said coupling agent is imidazolium trifluoroacetate, imidazolium triflate, imidazolium perchlorate, imidazolium acetate, imidazolium tosylate or imidazolium nitrate.

29. The method of claim 27 wherein said coupling agent is pyridinium trifluoroacetate, pyridinium triflate, pyridinium perchlorate, pyridinium acetate, pyridinium tosylate or pyridinium nitrate.

30. The method of claim 18 wherein said 2'-substituent is attached to the 2'-position through an oxygen atom.

31. The method of claim 30 wherein said 2'-substituent is O-alkyl, O-PG, O(CH₂)ₙOCH₃, or O[(CH₂)ₙO]ₘCH₃, wherein PG is a hydroxyl protecting group and n and m are from 1 to 10.

32. The method of claim 31 wherein said 2'-substiuent is 2'-O-alkyl.

33. The method of claim 32 wherein said alkyl group is C₁ to C₁₂ alkyl.

34. The method of claim 33 wherein said alkyl group is methyl.

35. The method of claim 31 wherein said 2'-substituent is O(CH₂)ₙOCH₃ and n is from 1 to 3.

36. The method of claim 35 wherein said 2'-substituent is O(CH₂)₂OCH₃.

37. The method of claim 18 wherein said activated phosphate moiety comprises a B-cyanoethyl protecting group.

38. The method of claim 18 wherein said activated phosphate moiety comprises an acetoxy phenoxy ethyl group.

## Patentansprüche

1. Verfahren zur Herstellung einer Internukleotid-Phosphorothioatverknüpfung mit angereichertem Rp-Enantiomer zwischen einem Synthon mit einer Hydroxylgruppierung an der 5`-Position und einem 2`-substituierten Nukleosid mit einer aktivierten Phosphatgruppierung an der 3'-Position, wobei man:
ein Kupplungsreagens mit einem pKa-Wert im Bereich von 3,3 bis 4,5 auswählt;
das Synthon an das 2'-substituierte Nukleosid in Gegenwart des Kupplungsreagens unter Bildung einer Phosphit-Zwischenstufe kuppelt; und
die Phosphit-Zwischenstufe mit Phenylacetyldisulfid oxidiert.

2. Verfahren nach Anspruch 1, wobei das erste Synthon an einen Träger gebunden ist.

3. Verfahren nach Anspruch 1, wobei es sich bei dem Synthon mit einer Hydroxylgruppierung an der 5'-Position um ein Desoxyribonukleotid, ein Ribonukleotid, ein 2'-geschütztes Ribonukleotid oder ein 2'-substituiertes Ribonukleotid handelt.

4. Verfahren nach Anspruch 1, wobei das Kupplungsreagens einen pKa-Wert im Bereich von 3,4 bis 4,4 aufweist.

5. Verfahren nach Anspruch 1, wobei das Kupplungsreagens einen pKa-Wert im Bereich von 3,5 bis 4,3 aufweist.

6. Verfahren nach Anspruch 1, wobei das Kupplungsreagens einen pKa-Wert im Bereich von 3,6 bis 4,3 aufweist.

7. Verfahren nach Anspruch 1, wobei das Kupplungsreagens einen pKa-Wert im Bereich von 3,7 bis 4,3 aufweist.

8. Verfahren nach Anspruch 1, wobei es sich bei dem Kupplungsreagens um 5-(Ethylthio)-1H-tetrazol handelt.

9. Verfahren nach Anspruch 1, wobei der 2`-Substi-tuent über ein Sauerstoffatom an die 2'-Position gebunden ist.

10. Verfahren nach Anspruch 9, wobei es sich bei dem 2'-Substituenten um O-Alkyl, O-PG, O(CH₂)ₙOCH₃ oder O[(CH₂)ₙO]ₘCH₃ handelt, wobei PG für eine Hydroxyl-Schutzgruppe steht und n bzw. m gleich 1 bis 10 ist.

11. Verfahren nach Anspruch 10, wobei es sich bei dem 2`-Substituenten um 2'-O-Alkyl handelt.

12. Verfahren nach Anspruch 11, wobei es sich bei der Alkylgruppe um C₁- bis C₁₂-Alkyl handelt.

13. Verfahren nach Anspruch 12, wobei es sich bei der Alkylgruppe um Methyl handelt.

14. Verfahren nach Anspruch 10, wobei es sich bei dem 2'-Substituenten um O(CH₂)ₙOCH₃ handelt und n gleich 1 bis 3 ist.

15. Verfahren nach Anspruch 9, wobei es sich bei dem 2'-Substituenten um O(CH₂)₂OCH₃ handelt.

16. Verfahren nach Anspruch 1, wobei die aktivierte Phosphatgruppierung eine B-Cyanoethyl-Schutzgruppe umfasst.

17. Verfahren nach Anspruch 1, wobei die aktivierte Phosphatgruppierung eine Acetoxyphenoxyethylgruppe umfasst.

18. Verfahren zur Herstellung einer Internukleotid-Phosphorothioatverknüpfung mit angereichertem Sp-Enantiomer zwischen einem Synthon mit einer Hydroxylgruppierung an der 5`-Position und einem 2`-substituierten Nukleosid mit einer aktivierten Phosphatgruppierung an der 3'-Position, wobei man:
ein Kupplungsreagens mit einem pKa-Wert im Bereich von 6,0 bis 8,0 auswählt;
das Synthon an das 2`-substituierte Nukleosid in Gegenwart des Kupplungsreagens kuppelt; und das Enantiomerenverhältnis misst.

19. Verfahren nach Anspruch 18, wobei das erste Synthon an einen Träger gebunden ist.

20. Verfahren nach Anspruch 18, wobei es sich bei dem Synthon mit einer Hydroxylgruppierung an der 5'-Position um ein Desoxyribonukleotid, ein Ribonukleotid, ein 2'-geschütztes Ribonukleotid oder ein 2'-substituiertes Ribonukleotid handelt.

21. Verfahren nach Anspruch 18, wobei das Kupplungsreagens einen pKa-Wert im Bereich von 6,5 bis 7,5 aufweist.

22. Verfahren nach Anspruch 18, wobei das Kupplungsreagens einen pKa-Wert im Bereich von 6,2 bis 7,3 aufweist.

23. Verfahren nach Anspruch 18, wobei das Kupplungsreagens einen pKa-Wert im Bereich von 6,4 bis 7,1 aufweist.

24. Verfahren nach Anspruch 18, wobei das Kupplungsreagens einen pKa-Wert im Bereich von 6,5 bis 7,0 aufweist.

25. Verfahren nach Anspruch 18, wobei das Kupplungsreagens einen pKa-Wert im Bereich von 6,7 bis 6,9 aufweist.

26. Verfahren nach Anspruch 18, wobei es sich bei dem Kupplungsreagens um ein Imidazolium- oder Pyridinium-Derivat handelt.

27. Verfahren nach Anspruch 26, wobei es sich bei dem Kupplungsreagens um ein Imidazolium- oder Pyridiniumsalz handelt.

28. Verfahren nach Anspruch 27, wobei es sich bei dem Kupplungsreagens um Imidazoliumtrifluoracetat, Imidazoliumtriflat, Imidazoliumperchlorat, Imidazoliumacetat, Imidazoliumtosylat oder Imidazoliumnitrat handelt.

29. Verfahren nach Anspruch 27, wobei es sich bei dem Kupplungsreagens um Pyridiniumtrifluoracetat, Pyridiniumtriflat, Pyridiniumperchlorat, Pyridiniumacetat, Pyridiniumtosylat oder Pyridiniumnitrat handelt.

30. Verfahren nach Anspruch 18, wobei der 2'-Substituent über ein Sauerstoffatom an die 2`-Position gebunden ist.

31. Verfahren nach Anspruch 30, wobei es sich bei dem 2'-Substituenten um O-Alkyl, O-PG, O(CH₂)ₙOCH₃ oder O[(CH₂)ₙO]ₘCH₃ handelt, wobei PG für eine Hydroxyl-Schutzgruppe steht und n bzw. m gleich 1 bis 10 ist.

32. Verfahren nach Anspruch 31, wobei es sich bei dem 2`-Substituenten um 2'-O-Alkyl handelt.

33. Verfahren nach Anspruch 32, wobei es sich bei der Alkylgruppe um C₁- bis C₁₂-Alkyl handelt.

34. Verfahren nach Anspruch 33, wobei es sich bei der Alkylgruppe um Methyl handelt.

35. Verfahren nach Anspruch 31, wobei es sich bei dem 2'-Substituenten um O(CH₂)ₙOCH₃ handelt und n gleich 1 bis 3 ist.

36. Verfahren nach Anspruch 35, wobei es sich bei dem 2'-Substituenten um O(CH₂)₂OCH₃ handelt.

37. Verfahren nach Anspruch 18, wobei die aktivierte Phosphatgruppierung eine B-Cyanoethyl-Schutzgruppe umfasst.

38. Verfahren nach Anspruch 18, wobei die aktivierte Phosphatgruppierung eine Acetoxyphenoxyethylgruppe umfasst.

## Revendications

1. Procédé pour préparer un lieur phosphorothioate internucléotidique enrichi en énantiomère Rp entre un synthon ayant un fragment hydroxyle à la position 5' et un nucléoside substitué en 2' ayant un fragment phosphate activé à la position 3' comprenant :
la sélection d'un agent de couplage ayant un pKa dans la plage de 3,3 à 4,5 ;
le couplage dudit synthon audit nucléoside substitué en 2' en présence dudit agent de couplage pour former un intermédiaire phosphite ; et
l'oxydation dudit intermédiaire phosphite avec du disulfure de phénylacétyle.

2. Procédé de la revendication 1, ledit premier synthon étant lié à un support.

3. Procédé de la revendication 1, le synthon ayant un fragment hydroxyle à la position 5' étant un désoxyribonucléotide, un ribonucléotide, un ribonucléotide protégé en 2' ou un ribonucléotide substitué en 2'.

4. Procédé de la revendication 1, ledit agent de couplage ayant un pKa dans la plage de 3,4 à 4,4.

5. Procédé de la revendication 1, ledit agent de couplage ayant un pKa dans la plage de 3,5 à 4,3.

6. Procédé de la revendication 1, ledit agent de couplage ayant un pKa dans la plage de 3,6 à 4,3.

7. Procédé de la revendication 1, ledit agent de couplage ayant un pKa dans la plage de 3,7 à 4,3.

8. Procédé de la revendication 1, ledit agent de couplage étant 5-(éthylthio)-1H-tétrazole.

9. Procédé de la revendication 1, ledit substituant en 2' étant lié à la position 2' par l'intermédiaire d'un atome d'oxygène.

10. Procédé de la revendication 9, ledit substituant en 2' étant O-alkyle, O-PG, O(CH₂)ₙOCH₃, ou O [(CH₂)ₙO]ₘCH₃, où PG est un groupe protecteur d'hydroxyle et n et m sont de 1 à 10.

11. Procédé de la revendication 10, ledit substituant en 2' étant 2'-O-alkyle.

12. Procédé de la revendication 11, ledit groupe alkyle étant alkyle en C₁ à C₁₂.

13. Procédé de la revendication 12, ledit groupe alkyle étant méthyle.

14. Procédé de la revendication 10, ledit substituant en 2' étant O(CH₂)ₙOCH₃ et n étant de 1 à 3.

15. Procédé de la revendication 9, ledit substituant en 2' étant O(CH₂)₂OCH₃.

16. Procédé de la revendication 1, ledit fragment phosphate activé comprenant un groupe protecteur β-cyanoéthyle.

17. Procédé de la revendication 1, ledit fragment phosphate activé comprenant un groupe acétoxyphénoxyéthyle.

18. Procédé pour préparer un lieur phosphorothioate internucléotidique enrichi en énantiomère Sp entre un synthon ayant un fragment hydroxyle à la position 5' et un nucléoside substitué en 2' ayant un fragment phosphate activé à la position 3' comprenant :
la sélection d'un agent de couplage ayant un pKa dans la plage de 6,0 à 8,0 ;
le couplage dudit synthon audit nucléoside substitué en 2' en présence dudit agent de couplage ; et la mesure du rapport énantiomérique.

19. Procédé de la revendication 18, ledit synthon étant lié à un support.

20. Procédé de la revendication 18, le synthon ayant un fragment hydroxyle à la position 5' étant un désoxyribonucléotide, un ribonucléotide, un ribonucléotide protégé en 2' ou un ribonucléotide substitué en 2'.

21. Procédé de la revendication 18, ledit agent de couplage ayant un pKa dans la plage de 6,5 à 7,5.

22. Procédé de la revendication 18, ledit agent de couplage ayant un pKa dans la plage de 6,2 à 7,3.

23. Procédé de la revendication 18, ledit agent de couplage ayant un pKa dans la plage de 6,4 à 7,1.

24. Procédé de la revendication 18, ledit agent de couplage ayant un pKa dans la plage de 6,5 à 7,0.

25. Procédé de la revendication 18, ledit agent de couplage ayant un pKa dans la plage de 6,7 à 6,9.

26. Procédé de la revendication 18, ledit agent de couplage étant un dérivé d'imidazolium ou de pyridinium.

27. Procédé de la revendication 26, ledit agent de couplage étant un sel d'imidazolium ou de pyridinium.

28. Procédé de la revendication 27, ledit agent de couplage étant le trifluoroacétate d'imidazolium, le triflate d'imidazolium, le perchlorate d'imidazolium, l'acétate d'imidazolium, le tosylate d'imidazolium ou le nitrate d'imidazolium.

29. Procédé de la revendication 27, ledit agent de couplage étant le trifluoroacétate de pyridinium, le triflate de pyridinium, le perchlorate de pyridinium, l'acétate de pyridinium, le tosylate de pyridinium ou le nitrate de pyridinium.

30. Procédé de la revendication 18, ledit substituant en 2' étant lié à la position 2' par l'intermédiaire d'un atome d'oxygène.

31. Procédé de la revendication 30, ledit substituant en 2' étant O-alkyle, O-PG, O(CH₂)ₙOCH₃, ou O[(CH₂)ₙO]mCH₃, où PG est un groupe protecteur d'hydroxyle et n et m sont de 1 à 10.

32. Procédé de la revendication 31, ledit substituant en 2' étant 2'-O-alkyle.

33. Procédé de la revendication 32, ledit groupe alkyle étant alkyle en C₁ à C₁₂.

34. Procédé de la revendication 33, ledit groupe alkyle étant méthyle.

35. Procédé de la revendication 31, ledit substituant en 2' étant O(CH₂)ₙOCH₃ et n étant de 1 à 3.

36. Procédé de la revendication 35, ledit substituant en 2' étant O(CH₂)₂OCH₃.

37. Procédé de la revendication 18, ledit fragment phosphate activé comprenant un groupe protecteur β-cyanoéthyle.

38. Procédé de la revendication 18, ledit fragment phosphate activé comprenant un groupe acétoxyphénoxyéthyle.
